(19) Europäisches Patentamt
European Patent Office
Office européen des brevets

(11) Numéro de publication : 0 337 885 B1

(12) # FASCICULE DE BREVET EUROPEEN

(45) Date de publication du fascicule du brevet : **17.03.93 Bulletin 93/11**

(51) Int. Cl.[5] : **C07D 311/58,** A61K 31/35

(21) Numéro de dépôt : **89401017.2**

(22) Date de dépôt : **13.04.89**

(54) **Nouveaux dérivés triéniques de structure chroménique, leurs procédés de préparation et les compositions pharmaceutiques qui les contiennent.**

(30) Priorité : **13.04.88 FR 8804871**

(43) Date de publication de la demande : **18.10.89 Bulletin 89/42**

(45) Mention de la délivrance du brevet : **17.03.93 Bulletin 93/11**

(84) Etats contractants désignés : **AT BE CH DE ES FR GB GR IT LI LU NL SE**

(56) Documents cités : **GB-A- 2 188 634**

(73) Titulaire : **ADIR ET COMPAGNIE**
**1 rue Carle Hébert**
**F-92415 Courbevoie Cédex (FR)**

(72) Inventeur : **Brion, Jean Daniel**
**76 Rue du Château**
**F-95320 Saint Leu La Forêt (FR)**
Inventeur : **Le Baut, Guillaume**
**5 Rue de la Baugerie**
**F-44230 Saint Sébastien sur Loire (FR)**
Inventeur : **Ducrey, Patrick**
**7 Rue du Docteur Zamenhof**
**F-92500 Rueil Malmaison (FR)**
Inventeur : **Piessard-Robert, Sylvie**
**11 Boulevard des Martyrs Nantais**
**F-44200 Nantes (FR)**
Inventeur : **Cudennec, Claude**
**22 bis Avenue de Circourt**
**F-78170 La Celle St. Cloud (FR)**
Inventeur : **Seurre, Geneviève**
**3 Impasse de l'Enfant Jésus**
**F-75015 Paris (FR)**

EP 0 337 885 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés doués de propriétés anticancéreuses appartenant à la famille des rétinoïdes, leur procédé de préparation et les compositions pharmaceutiques qui les contiennent.

On connait de nombreuses substances de la famille des rétinoïdes réputées pour leurs propriétés anticancéreuses. Les brevets US 4,054,589; US 4,106,681 ; US 4,137,246 ; US 4,165,103 ; US 4,169,100 ; US 4,171,318 ; US 4,224,244 ; BE 861982 ; FR 2 556 348 ; EP 111124 décrivent des dérivés de la famille des rétinoïdes dont la chaîne tétraénique est portée par une structure monocyclique cyclohexénique ou benzénique.

Plus particulièrement le brevet US 4,105,681 décrit l'étrétinate utilisé en thérapeutique et dont les propriétés anticancéreuses sont aujourd'hui connues.

Le brevet US 4,321,209 ainsi que les documents de priorité DT 2 542 600 et DT 2 542 601 décrivent des dérivés dont la chaîne triénique est fixée sur une structure monocyclique aromatique.

On peut également citer le brevet GB-A-2188634 qui décrit des dérivés dont la chaîne triénique est fixée sur une structure bicyclique dérivée du chromène en position 6.

Les besoins de la thérapeutique exigent le développement constant de nouveaux anticancéreux dans le double but d'obtenir des molécules à la fois plus actives mais également moins toxiques.

Les dérivés de la présente invention possèdent une structure originale constituée d'une chaîne triénique fixée sur une structure bicyclique de type chromène que l'on rencontre dans de nombreuses substances naturelles éventuellement substituée sur le noyau aromatique. L'originalité de la structure des composés de la présente invention permet l'obtention de propriétés pharmacologiques particulièrement intéressantes puisque se révélant dans les tests étudiés, supérieures à celle de l'étrétinate. En outre, leur toxicité est particulièrement faible puisqu'il est apparu que ces dérivés n'entraînaient pas de toxicité hypervitaminique A contrairement à l'étrétinate.

Plus particulièrement la présente invention a pour objet des produits de formule générale (I) :

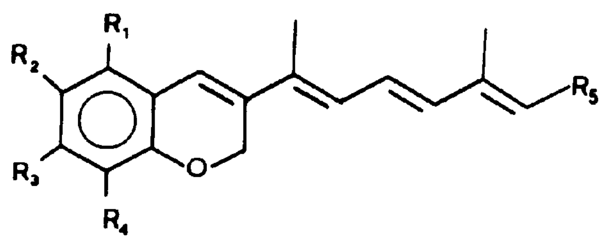

(I)

dans laquelle :

$R_1$, $R_2$, $R_3$, $R_4$ identiques ou différents représentent un atome d'hydrogène, un atome d'halogène, un groupement alcoyl inférieur, alkényl inférieur, alcoyloxy inférieur ou alkényloxy inférieur éventuellement substitués par un ou plusieurs atomes d'halogène,

$R_5$ représente un carboxyle, alcoyloxy inférieur carbonyle, alkényloxy inférieur carbonyle, alkynyloxy inférieur carbonyle,

leurs isomères, stéréoisomères, diastéréoisomères ainsi que leurs sels d'addition à une base pharmaceutiquement acceptable.

Les composés préférés possèdent la configuration (E,E,E) au niveau de la chaîne triénique.

Parmi les bases susceptibles de salifier les composés de formule (I) dans lesquels $R_5$ représente un groupement carboxyle, on peut citer à titre d'exemple, les hydroxydes de sodium, potassium, calcium, ou d'aluminium, les carbonates de métaux alcalins ou alcalino terreux ou des bases organiques comme la triéthylamine, la benzylamine, la diéthanolamine, la tert.butylamine, la dicyclohexylamine, l'arginine etc...

Par radicaux alcoyle inférieur, alkényl inférieur, alcoyloxy inférieur, alkényloxy inférieur, alkynyloxy inférieur, on entend des groupements linéaires ou ramifiés comprenant entre 1 et 6 atomes de carbone.

La présente invention s'étend également à un procédé de préparation des dérivés de formule (I) caractérisé en ce que l'on condense par chauffage à reflux du solvant un dérivé de formule (II) :

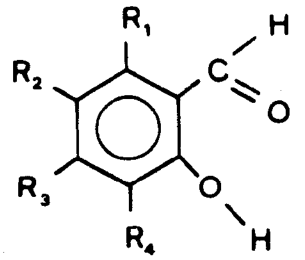

(II)

dans lequel $R_1$, $R_2$, $R_3$ et $R_4$ ont la même signification que dans la formule (I)

avec l'oxo - 3 butène - 1 de formule (III) :

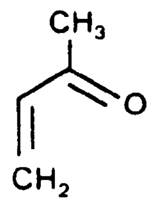

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellemnt choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique évaporation du solvant et purification du résidu par cristallisation ou distillation ou chromatographie sur colonne de silice ou d'alumine,

à un dérivé de formule (IV) :

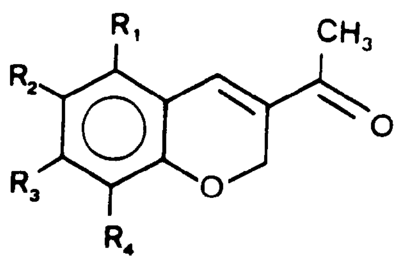

(IV)

dans laquelle $R_1$, $R_2$, $R_3$ et $R_4$ ont la même signification que dans la formule (I) lequel est soumis,

* soit à hydrogénation catalytique, après dissolution dans un solvant organique convenable préférentiellement choisi parmi les alcools aliphatiques inférieurs, préférentiellement en présence d'un agent alcalin tel que l'hydroxyde de sodium, hydrogénation réalisée préférentiellement par un hydrure mixte de métal alcalin tel que le borohydrure de sodium pour conduire,

après une extraction par un solvant organique convenable tel que l'éther diéthylique, le dichlorométhane ou le chloroforme, éventuellement précédée de l'évaporation du milieu réactionnel, lavage puis évaporation de la phase organique et purification à un dérivé de formule (V) :

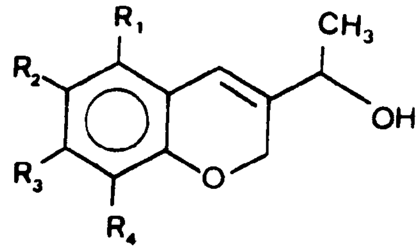

(V)

dans laquelle $R_1$, $R_2$, $R_3$ et $R_4$ ont la même signification que précédemment, lequel est soumis à l'action d'un sel de triphényl phosphonium dans un solvant préférentiellement choisi parmi les alcools aliphatiques inférieurs à température ambiante et sous agitation pour conduire, après évaporation du solvant et purification par chromatographie sur gel de silice et cristallisation dans un solvant ou un mélange de solvants appropriés,

à un dérivé de formule (VI) :

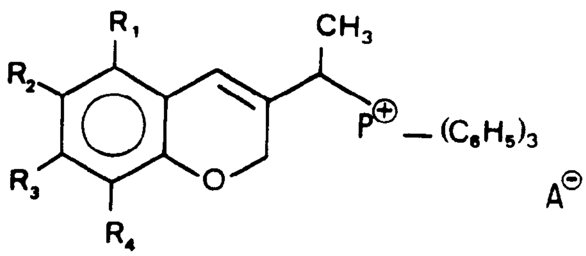

(VI)

dans lequel $A^-$ représente l'anion d'un hydracide et $R_1$, $R_2$, $R_3$ et $R_4$ ont la même signification que précédemment,

que l'on soumet dans un solvant approprié préférentiellement sous atmosphère inerte et en présence de n butyllithium dans l'hexane à l'action d'un aldéhyde diénique de stéréochimie bloquée sous forme d'un complexe tricarbonyl fer de formule (VII) selon la réaction décrite par WITTIG (Ber 1954, 87, 1318 et Ber 1955, 88, 1654) :

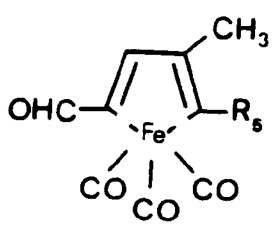

(VII)

dans lequel $R_5$ a la même signification que dans la formule (I),

à une température préférentiellement comprise entre -5°C et -30°C, préférentiellement comprise entre -15°C et -20°C, pour conduire, après extraction par un solvant organique approprié, lavage et évaporation de la phase organique, à un complexe intermédiaire, qui, après purification par chromatographie est soumis, à reflux du solvant organique choisi pour cette étape, à l'action du N oxyde de triméthylamine, pour conduire, après refroidissement, extraction par un solvant organique approprié, lavage et évaporation de la phase organique, et chromatographie sur colonne,

à un composé de formule (I),

* soit à l'action du bromoacétate d'éthyle en présence de zinc, suivie d'une hydrolyse extemporanée du milieu réactionnel, extraction par un solvant organique approprié préférentiellement choisi parmi acétate d'éthyle, chlorure de méthylène, chloroforme, éther diéthylique, puis à l'action de l'oxychlorure de phosphore dans un solvant organique approprié, évaporation du milieu réactionnel et nouvelle extraction par

un solvant préférentiellement choisi parmi acétate d'éthyle, chlorure de méthylène, chloroforme, éther diéthylique pour conduire après une éventuelle chromatographie au gel de silice à un dérivé de formule (VIII):

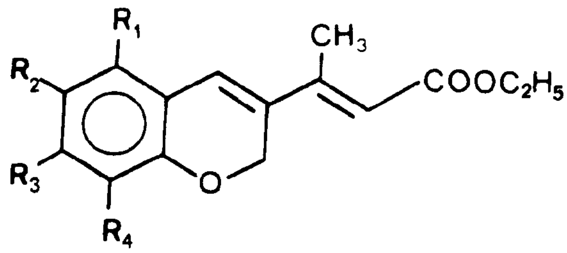

(VIII)

dans laquelle $R_1$, $R_2$, $R_3$ et $R_4$ ont la même signification que dans la formule (I),

que l'on soumet à basse température préférentiellement comprise entre - 50 et 0 °C, préférentiellement comprise entre - 25 et 5 °C dans un solvant préférentiellement choisi parmi éther diéthylique, éther diisopropylique, tétrahydrofuranne à l'action d'hydrure de lithium aluminium pour conduire à un dérivé de formule (IX) :

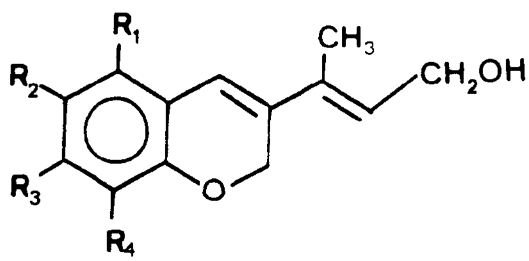

(IX)

dans laquelle $R_1$, $R_2$, $R_3$, $R_4$ ont la même signification que dans la formule (I),

que l'on soumet à oxydation par le dioxyde de manganèse pour conduire après chromatographie sur gel de silice et éventuelle cristallisation à un dérivé de formule (X) :

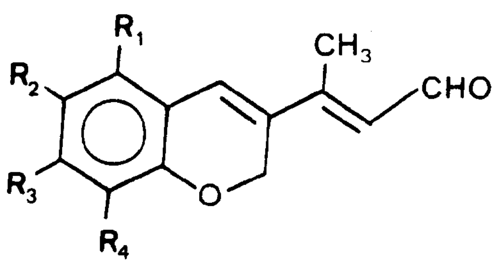

(X)

dans laquelle $R_1$, $R_2$, $R_3$, $R_4$ ont la même signification que dans la formule (I),

que l'on soumet à l'action d'un dérivé de formule (XI) selon la réaction de WADSWORTH-EMMONS (J. Am. Chem. Soc. 1961, 83, 1733) :

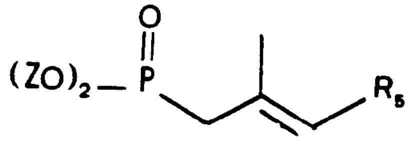

(XI)

dans laquelle $R_5$ a la même signification dans la formule (I), et Z un groupement alkyle inférieur,

à température ambiante pour conduire, après extraction par un solvant organique approprié, évaporation, purification par chromatographie sur colonne de silice à un composé de formule (I),

que l'on peut si on le désire,

- soit lorsque $R_5$ représente un groupement carboxyle, salifier par une base pharmaceutiquement acceptable.

- soit séparer en ses stéréoisomères par une technique de cristallisation ou de chromatographie puis, si on le désire, lorsque R5 représente un groupement carboxyle, salifier par une base pharmaceutiquement acceptable.

Les composés de formule (I) possèdent des propriétés pharmacologiques très intéressantes. Ils inhibent de façon importante la croissance de la lignée L 1210 chez la souris, ils ont une activité sur la différenciation des cellules de la lignée HL 60, et fait particulièrement intéressant, pour leur utilisation en thérapeutique, ne semblent pas entraîner de toxicité hypervitaminique A, induite par l'étrétinate et dont les symptômes sont la perte de poids rapide, l'alopécie et la fragilisation des os.

Les composés selon la présente invention trouvent donc une utilisation en thérapeutique et tant qu'agents antitumoraux, pour le traitement ou la prophylaxie, des néoplasmes bénins ou malins, ainsi que dans les indications classiques des rétinoïdes, tels que les désordres cutanés (acné, psoriasis) ainsi que les troubles dégénératifs et/ou inflammation des muqueuses.

La présente invention a également pour objet les compositions pharmaceutiques contenant les produits de formule (I), ou un de leurs sels d'addition à une base pharmaceutiquement acceptable lorsque $R_5$ représente un groupement carboxyle, seul ou en combinaison avec un ou plusieurs excipients ou véhicules inertes, non toxiques, pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, pommades, gels dermiques, les préparations injectables ou buvables, les aérosols, les gouttes oculaires ou nasales...etc...

La posologie utile varie selon l'âge et le poids du patient, la voie d'administration, la nature de l'indication thérapeutique et des traitements éventuellement associés et s'échelonne entre 0,1 et 200 mg par jour.

Les exemples suivants illustrent l'invention mais ne la limitent en aucune façon.

Les préparations indiquées ci-dessous ne permettent pas d'obtenir des produits de l'invention. Elles permettent néanmoins l'obtention d'intermédiaires utiles dans la synthèse des produits de l'invention.

**PREPARATION 1 : TRICARBONYL [$\eta$ 1 - 4 (ETHOXYCARBONYL - 1 METHYL - 2 OXO - 5 PENTADIENE - 1 (E), 3 (E) yl] FER**

**STADE A : DIMETHOXY - 4,4 METHYL - 3 BUTENE - 2 OATE D'ETHYLE**

Un mélange de 50 mmole de diméthoxy - 1,1 acétone et de 60 mmole de (diéthoxy - phosphoryl) - 2 acétate d'éthyle est ajouté en 1 heure à une suspension de 125 mmole de carbonate de potassium dans 10 ml d'eau en agitant énergiquement et en maintenant la température entre 20 et 30°C. Une fois l'addition terminée, l'agitation est poursuivie 24 heures à température ambiante puis l'insoluble est éliminé par filtration et lavé à l'éther diéthylique. La phase organique est séparée et lavée par une solution saturée de chlorure de sodium jusqu'à neutralité. Après séchage et évaporation du solvant, le produit est purifié par distillation sous vide qui livre un mélange d'isomère (E + Z) sous forme huileuse.

**STADE B : METHYL - 3 OXO - 4 BUTENE 2 (E) OATE D'ETHYLE**

50 mmole de diméthoxy - 4,4 méthyl - 3 butène - 2 oate d'éthyle (E + Z) sont placées en milieu acétique

tamponné (3 g d'acétate de sodium, 1,5 ml d'eau, 30 ml d'acide acétique). Le milieu réactionnel est chauffé à 100°C pendant 5 heures puis les solvants sont éliminés sous vide. Après reprise du résidu par de l'eau et addition de 100 ml d'éther diéthylique, la phase organique est séparée et lavée par 100 ml d'une solution saturée de chlorure de sodium. Après séchage et évaporation des solvants, la purification est assurée par distillation sous vide et livre le stéréoisomère E.
Rendement : 85 %  $Eb_1$ = 44 - 46 °C

**STADE C : METHYL - 3 OXO - 6 HEXADIENE - 2 (E), 4 (E) OATE D'ETHYLE**

Sous agitation vigoureuse et atmosphère d'azote, une solution de 50 mmole de méthyle - 3 oxo - 4 butène - 2 (E) oate d'éthyle dans 25 ml de benzène est ajoutée en 10 minutes à une suspension benzénique (100 ml) de 50 mmole de formylméthylènetriphénylphosphorane, la température étant maintenue vers 0°C. Après retour à température ambiante, le milieu réationnel est chauffé au reflux pendant 6 heures. Après refroidissement, le milieu est versé sur un mélange eau-éther éthylique puis la phase organique séparée et séchée. L'évaporation des solvants est suivie d'une chromatographie sur colonne de gel de silice ($SiO_2$ : 70 - 230 Mesh, éluant : $CH_2CL_2$) qui livre une huile (mélange d'isomères) dont est séparé par cristallisation fractionnée l'isomère (E,E).
Rendement : 65 %.

**STADE D : TRICARBONYL [η 1 - 4 (ETHOXY - CARBONYL - 1 METHYL - 2 OXO - 5 PENTADIENE - 1 (E), 3 (E), YL] FER**

Une solution de 10 mmole de méthyl - 3 oxo - 6 hexadiène - 2 (E), 4 (E) oate d'éthyle dans 75 ml de toluène est dégazée par un courant d'azote pendant 30 minutes avant l'addition de 34 ml (21 mmole) de pentacarbonylfer, puis irradiée. La photolyse est réalisée, dans une cellule en pyrex à refroidissement par circulation d'eau, par une lampe Philips HPK 125W pendant 18 heures à une température voisine de 35°C. Après évaporation du solvant, le résidu est purifié par une chromatographie sur colonne de gel de silice ($SiO_2$ : 70 - 23° Mesh, éluant : $CH_2Cl_2$) qui livre une huile jaune. (Rendement : 85 %) On obtient de la même façon les tricarbonyl [n 1 - 4 (alcoxy - carbonyl - 1 méthyl - 2 oxo - 5 pentadiène - 1 (E), 3 (E) yl] fer en utilisant le (diéthoxy phosphoryl) - 2 acétate d'alkyle approprié.

**PREPARATION 2 : DIETHOXYPHOSPHORYL - 4 METHYL - 3 BUTENE - 2 (E) OATES D'ETHYLE (E)**

**STADE A : HYDROXY - 4 METHYL - 3 BUTENE - 2 (E) OATE D'ETHYLE**

Sous agitation et à une température inférieure à 20°C, 0,125 mmole de méthyl - 3 oxo - 4 butène (E) oate d'éthyle obtenu au stade B, préparation 1, dissoute dans 100 ml d'éthanol, est réduite par une solution hydroalcoolique de 4,72 g (125 mmole) de borohydrure de sodium. L'agitation est poursuivie 1 heure après la fin de l'addition, puis le milieu réactionnel est filtré et l'insoluble lavé à l'éther diéthylique. Après élimination des solvants, le résidu est repris à l'eau et extrait par 3 x 100 ml d'éther diéthylique. Après séchage et évaporation des solvants, l'huile est purifiée par passage sur colonne de gel de silice (éluant : dichlorométhane, $SiO_2$ : 70 - 230 mesh).
Rendement : 76 % Huile

**STADE B : BROMO - 4 METHYL - 3 BUTENE - 2 (E) OATE D'ETHYLE (E)**

A l'abri de l'humidité, sous agitation et à froid (0°-5°C), 10,5 g (39 mmole) de tribromure de phosphore sont ajoutés goutte à goutte (10 minutes) à une solution éthérée (75 ml) de 100 mmole d'hydroxy - 4 méthyl - 3 butène - 2 (E) oate d'éthyle obtenue précédemment. Après un temps de contact de 1h30, le milieu réactionnel est versé sur l'eau. La phase organique est séparée est lavée par une solution saturée d'hydrogénocarbonate de sodium jusqu'à neutralité. Après séchage et évaporation du solvant, le résidu et chromatographié sur colonne de gel de silice (éluant : dichlorométhane : $SiO_2$ : 70 - 230 mesh).
Rendement : 91 %  Huile

**STADE C : DIETHOXYPHOSPHORYL - 4 METHYL - 3 BUTENE - 2 (E) OATE D'ETHYLE**

50 mmole de bromo - 4 méthyl - 3 butène - 2 (E) oate d'éthyle sont chauffées à 130 - 140°C à l'abri de l'humidité. Après avoir cessé le chauffage, 50 mmole de triéthylphosphite sont additionnées au goutte à goutte de manière à maintenir la température vers 130°C ; le bromoéthane formé s'élimine au fur et à mesure. Une

fois l'addition terminée, la réaction est complétée par un chauffage de 30 minutes à 150 - 160°C. Une distillation sous vide permet de recueillir le produit attendu.
Rendement : 94 % $E_2$ : 150 - 152°C

Les diéthoxy phosphoryl - 4 méthyl - 3 butène - 2 (E) oates d'alkyle s'obtiennent de façon identique en utilisant, au stade A le méthyl - 3 oxo - 4 butène (E) oate d'alkyle correspondant au produit attendu.

**EXEMPLE 1 : (DIMETHYL - 6,8 2H - CHROMENYL - 3) - 7 METHYL - 3 OCTATRIENE 2,4,6 OATE D'ETHYLE (E,E,E)**

**STADE A : DIMETHYL - 6,8 ACETYL - 3 2H - CHROMENE**

A une suspension de 0,14 g (1 mmole) de carbonate de potassium dans 50 ml de butanone - 2, additionner à température ambiante 1,50 g (10 mmole) d'aldéhyde hydroxy - 2 diméthyl - 3,5 benzoïque ainsi que 0,66 g (10 mmole) d'oxo - 3 butène - 1. Porter à reflux sous agitation et maintenir à cette température pendant 90 minutes. Evaporer le milieu réactionnel au bain-marie sous vide, et reprendre le résidu par 100 ml d'eau et extraire à 3 reprises par 75 ml d'éther diéthylique. Réunir les phases organiques, laver jusqu'à neutralité par une solution aqueuse saturée de chlorure de sodium, sécher sur sulfate de sodium, filtrer et évaporer au bain-marie sous vide et chromatographie sur colonne de silice (70 - 230 mesh) en éluant par le dichlorométhane.
Rendement : 81 %.

**STADE B : DIMETHYL - 6,8 (HYDROXY - 1 ETHYL) - 3 2H - CHROMENE**

Dissoudre 2,02 g (10 mmole) de diméthyl - 6,8 acétyl - 3 2H - chromène obtenu au stade précédent dans 50 ml d'éthanol. Additionner en une fois sous agitation magnétique une solution de 0,38 g (10 mmole) de borohydrure de sodium dans 5 ml d'une solution aqueuse d'hydroxyde de sodium. Maintenir l'agitation 2 heures puis évaporer le mélange solvant. Reprendre le résidu par 50 ml d'eau et extraire à 3 reprises par 50 ml d'éther diéthylique. Laver la solution éthérée par une solution aqueuse saturée de chlorure de sodium jusqu'à neutralité des eaux de lavage, sécher et évaporer la phase organique.
Rendement : 86 %

**STADE C : BROMURE DE [(DIMETHYL - 6,8 2H - CHROMENYL - 3) - 1 ETHYL - 1] TRIPHENYLPHOSPHONIUM**

Placer sous atmosphère d'azote et à température ambiante 2,04 g (10 mmole) de diméthyl - 6,8 (hydroxy - 1 éthyl) - 3 2H chromène obtenus au stade précédent et 3,43 g (10 mmole) de bromhydrate de triphényl phosphonium dans 80 ml de méthanol. Agiter pendant 96 heures. Eliminer le résidu obtenu sur colonne de gel de silice (70 - 230 Mesh) en utilisant comme solvant d'élution le mélange chlorure de méthylène éthanol (95/5 v/v), cristalliser dans le benzène.
Rendement : 68%
Point de fusion : 164°C

**STADE D : (DIMETHYL - 6,8 2H - CHROMENYL - 3) - 7 METHYL - 3 OCTATRIENE - 2,4,6 OATE D'ETHYLE - (E,E,E)**

A une suspension de 5,29 g (10 mmole) de bromure de [(diméthyl - 6,8 2H - chroményl - 3) - 1 éthyl - 1] triphénylphosphonium dans 50 ml de tétrahydrofuranne, ajouter, en 10 minutes, sous agitation, à température ambiante et sous atmosphère inerte 70 ml d'une solution de n - butyllithium dans l'hexane (1,6 M). Poursuivre l'agitation 15 minutes puis refroidir à une température comprise entre -15°C et -20°C. Additionner 3,06 g (10 mmole) de tricarbonyl [η 1 - 4 - (éthoxycarbonyl - 1 méthyl - 2 oxo - 5 pentadiène - (1E, 3E)yl] fer, obtenu dans la préparation 1, en une fois. Maintenir le milieu réactionnel à température ambiante sous agitation pendant 18 heures. Verser dans 75 ml d'eau et extraire à trois reprises par 100 ml d'éther diéthylique. Réunir les phases organiques et laver jusqu'à neutralité par une solution aqueuse saturée de chlorure de sodium. Sécher sur sulfate de sodium, filtrer et évaporer l'éther diéthylique. Purifier par passage en chromatographie sur gel de silice.

Le produit obtenu est alors mis au contact de 11,15 g de dihydrate de triméthylamine N - oxyde dans 50 ml de chlorure de méthylène. Chauffer à reflux pendant 5 heures. Refroidir, verser dans 100 ml d'eau et extraire à trois reprises par 75 ml de dichlorométhane. Réunir les phases organiques, sécher sur sulfate de sodium, évaporer le solvant. Purifier le résidu par chromatographie sur gel de silice (70 - 230 Mesh) et recristalliser le

produit obtenu dans l'éther diisopropylique.
Rendement : 20 %
Point de fusion : 122°C

Analyse élémentaire :

| | | | | | | |
|---|---|---|---|---|---|---|
| Calculée : | C | : | 78,07 | H | : | 7,74 |
| Trouvée : | C | : | 77,41 | H | : | 8,14 |

Caractéristiques spectrales :

Infrarouge
Absorption à 1700, 1610, 1160 et 960 $cm^{-1}$

Résonance magnétique nucléaire (60 MHz) :

Solvant $CDCl_3$
δ = 1,30 ppm ; triplet 3H $CH_3$ ester J = 7Hz
δ = 2,00 à 2,45 ppm ; 4 singulets 4x3H 4$CH_3$ (chromène + chaîne)
δ = 4,25 ppm ; quadruplet 2H CH2 ester J = 7Hz
δ = 4,85 ppm ; 2H CH2 chromène
δ = 5,75 à 7,45 ppm ; 7H ; multiplet ; aromatiques et éthylèniques.

**EXEMPLE 2 : (2H - CHROMENYL - 3) - 7 METHYL - 3 OCTATRIENE - 2,4,6 OATE D'ETHYLE**

**STADE A : ACETYL - 3 2H - CHROMENE**

A une suspension de 0,14 g (1 mmole) de carbonate de potassium dans 50 ml de butanone - 2, additionner à température ambiante 1,22 g (10 mmole) d'aldéhyde salicylique ainsi que 0,66 g (10 mmole) d'oxo - 3 butène - 1. Porter à reflux sous agitation et maintenir cette température pendant 4 heures. Procéder ensuite comme dans l'exemple 1, stade A. La chromatographie finale est remplacée par une recristallisation dans l'hexane
Rendement : 57 %
Point de fusion : 58 - 60°C.

**STADE B : (HYDROXY - 1 ETHYL) - 3 2H - CHROMENE**

Procéder comme dans l'exemple 1, stade B en remplaçant le diméthyl - 6,8 acétyl - 3 2H - chromène par l'acétyl - 3 2H - chromène obtenu au stade précédent.
Rendement : 95 %

**STADE C : BROMURE DE [(2H) - CHROMENYL - 3) - 1 ETHYL] TRIPHENYLPHOSPHONIUM**

Procéder comme dans l'exemple 1, stade C en remplaçant le diméthyl - 6,8 (hydroxy - 1 éthyl) - 3 2H - chromène par l'(hydroxy - éthyl) - 3 2H - chromène.
Rendement : 198 %

**STADE D : (2H - CHROMENYL - 3) - 7 METHYL - 3 OCTATRIENE - 2,4,6 OATE D'ETHYLE**

Procéder comme dans l'exemple 1, stade D.
Solvant de cristallisation : dichlorométhane
Rendement : 30 %

**EXEMPLE 3 : (CHLORO - 6 METHYL - 8 2H CHROMENYL - 3) - 7 METHYL - 3 OCTATRIENE 2,4,6 OATE D'ETHYLE (E,E,E)**

**STADE A : CHLORO - 6 METHYL - 8 ACETYL - 3 2H CHROMENE**

En procédant comme dans l'exemple 1, stade A, mais en remplaçant l'aldéhyde hydroxy - 2 diméthyl - 3,5 benzoïque par l'aldéhyde hydroxy - 2 méthyl - 3 chloro - 5 benzoïque, on obtient le produit attendu que l'on purifie par une recristallisation supplémentaire dans le mélange éther isopropylique - éther de pétrole.
Point de fusion : 78 - 79°C.

**STADE B : (CHLORO -6 METHYL - 8 2H CHROMENYL - 3) - 3 BUTENE - 2 OATE D'ETHYLE**

Dans un ballon équipé d'un agitateur, d'une ampoule à brome, d'un réfrigérant et d'un thermomètre, est ajouté un volume de diméthoxyméthane suffisant pour recouvrir 0,108 at.g de zinc. On ajoute alors quelques gouttes de bromoacétate d'éthyle pur. La réaction est amorcée par un léger chauffage (à la flamme d'un briquet). La réaction est ensuite entretenue par addition goutte à goutte de 0,1 mmole de bromoacétate d'éthyle en solution dans du diméthoxyméthane anhydre. Le débit de l'addition est réglé de façon que la température ne dépasse par 40°C. Après addition, le milieu réactionnel est encore agité 30 minutes à température ambiante, puis 0,083 mmole d'acétyl - 3 chromène en solution dans du THF anhydre est ajoutée en une seule fois. Le milieu réactionnel est agité encore 15 minutes, puis hydrolysé par une solution saturée de chlorure d'ammonium. Après 3 extractions successives par l'acétate d'éthyle, les extraits organique sont séchés sur sulfate de magnésium et les solvants éliminés. Dissoudre 50 mmole du produit attendu dans 50 ml de benzène et ajouter 1 ml d'oxychlorure de phosphore. Porter au reflux pendant 1h30. Après refroidissement, le milieu réactionnel est versé dans 100 ml d'eau. La phase benzénique est décantée et la phase aqueuse extraite par 2 x 50 ml d'éther diéthylique. Après lavage des extraits organiques par une solution saturée d'hydrogénocarbonate de sodium jusqu'à neutralité, séchage et élimination des solvants, l'ester est purifié sur colonne de gel de silice ($SiO_2$) : 70 - 230 mesh ; éluant $CH_2Cl_2$).

Caractéristiques spectrales :

R.M.N. 1H $\delta$ : ppm (s : singulet ; t : triplet ; q : quadruplet : s.e. : signal étendu)
1,30 (3H, t, J = 7Hz, $OCH_2C\underline{H}_3$) ; 2,15 (3H, s, $CH_3$ en 8) ; 2,45 (3H, s.e., $C\underline{H}_3$)
4,20 (2H, q, J = 7Hz, $OC\underline{H}_2CH_3$) ; 4,95 (3H, s) ; 5,65 (1H, s.e., =

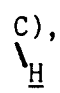

6,75, 6,90, 6,95, (3H, s.e., $H_4$, $H_5$ et $H_7$)

**STADE C : (CHLORO - 6 METHYL - 8 2H CHROMENYL - 3) - 3 BUTENE - 2 OL**

A l'abri de l'humidité, sous agitation et à froid (-15, -10°C), une solution de 20 mmole de l'ester obtenu au stade B, dans 70 ml d'éther diéthylique (ou de tétrahydrofuranne) est ajoutée, goutte à goutte, à 20 mmole d'hydrure de lithium et d'aluminium, en suspension dans le même solvant. Une fois la température revenue à l'ambiante, l'agitation est poursuivie pendant 2 heures puis l'excès d'hydrure est détruit par addition prudente d'acétate d'éthyle puis d'eau. Le milieu est ensuite acidifié (pH 2-3). Après séchage et évaporation des solvants, le produit brut est directement soumis à l'oxydation.
Rendement : 95 %

Caractéristiques spectrales :

Infrarouges :
3400 $\nu$ OH
1610 $\nu$ C = C
1235 $\nu$ C = O

**STADE D : (CHLORO - 6 METHYL - 8 2H CHROMENYL - 3) - 3 BUTENE - 2 (E) AL**

A froid (-15, -10°C) et sous agitation, une solution de 20 mmole de l'alcool choisi dans 50 ml de dichlorométhane est ajoutée lentement à 400 mmole de dioxyde de manganèse en suspension dans le minimum de dichlorométhane. L'addition terminée, l'agitation est poursuivie sous refroidissement pendant 1 à 3 heures, puis le milieu est filtré. Après évaporation du solvant, le produit obtenu est purifié sur colonne de gel de silice (SiO23 : 70 - 230 mesh) en éluant par du dichlorométhane.
Rendement : 20 %.

**STADE E : (CHLORO - 6 METHYL - 8 2H CHROMENYL - 3) - 7 METHYL-3 OCTATRIENE 2,4,6 OATE D'ETHYLE (E,E,E)**

Sous atmosphère d'azote et à température ambiante, à 1,20 g (4,54 mmole) de diéthoxy phosphoryl - 4 méthyl - 3 butène - 2 (E) oate d'éthyle obtenue dans la préparation 2, dissous dans 10 ml de THF anhydre, sont ajoutés 3 ml d'une solution hexanique de n-butyllithium (1,6 M). L'addition terminée, l'agitation est poursuivie pendant 15 minutes (coloration rougeâtre du milieu réactionnel), puis 0,91 g (4,54 mmole) d'aldéhyde chroménique obtenu au stade précédent, en solution dans 5 ml de THF est ajouté ; l'agitation est maintenue pendant 3 heures à température ambiante. Le milieu réactionnel est ensuite versé sur 100 ml d'eau et extrait par 3 x 25 ml d'éther diéthylique. Après séchage et évaporation des solvants le résidu est chromatographié sur colonne de gel de silice (2 chromatographies sur $SIO_2$ 70 - 230 mesh éluant : $CH_2Cl_2$ et une troisième sur $SiO_2$ 230 - 400 mesh ; éluant : $CH_2Cl_2$). Sont alors obtenus 400 mg d'un solide jaune, recristallisés dans le chlorure de méthylène.
Point de fusion : 107°C.

**EXEMPLE 4 : (2H - CHROMENYL - 3) - 7 METHYL - 3 OCTATRIENE - 2,4,6 OATE D'ETHYLE**

En procédant comme dans l'exemple 3, mais en remplaçant l'aldéhyde hydroxy - 2 méthyl - 3 chloro - 5 benzoïque par l'aldéhyde salicyclique, on obtient le produit du titre de l'exemple 2.

Au stade C, le temps de contact sera toutefois porté à 48 heures.
Point de fusion : 107°C.

**EXEMPLES 5 A 9 :**

En remplaçant dans l'exemple 1, stade A
∗ l'aldéhyde hydroxy - 2 diméthyl - 3,5 benzoïque par :
l'aldéhyde hydroxy - 2 - chloro - 5 méthyl - 3 benzolque, on obtient le (chloro - 6 méthyl - 8 2H chroményl - 3) - 7 méthyl - 3 octatriène 2,4,6 oate d'éthyle (E,E,E) **(EXEMPLE 5)**
- l'aldéhyde hydroxy - 2 diméthyl - 5,6 méthoxy - 4 benzoïque, on obtient le (diméthyl - 5,6 méthoxy - 7 2H - chroményl - 3) - 7 méthyl - 3 octatriène 2,4,6 oate d'éthyle (E,E,E) **(EXEMPLE 6)**
- l'aldéhyde hydroxy - 2 méthoxy - 4 benzoïque, on obtient le (méthoxy - 7 2H - chroményl - 3) - 7 méthyl - 3 octatriène 2,4,6 oate d'éthyle (E,E,E) **(EXEMPLE 7)**
- l'aldéhyde hydroxy - 2 méthoxy - 5 benzoïque, on obtient le (méthoxy - 6 2H - chroményl - 3) - 7 méthyl - 3 octatriène 2,4,6 oate d'éthyle (E,E,E) **(EXEMPLE 8)**
- l'aldéhyde hydroxy - 2 dichloro - 3,5 benzoïque, on obtient le (dichloro - 6,8 2H - chroményl - 3) - 7 méthyl - 3 octatriène 2,4,6 oate d'éthyle (E,E,E) **(EXEMPLE 9)**

Les composés des exemples 5 à 9 peuvent également être obtenus en utilisant le mode opératoire décrit pour l'exemple 3.

**ETUDE PHARMACOLOGIQUE**

**EXEMPLE 10 : INHIBITION DE CROISSANCE DE LA LIGNEE L1210**

La croissance de cette lignée leucémique de souris est appréciée par la capacité des cellules à incorporer la thymidine tritiée. Le taux d'incorporation est mesuré 48 heures après l'introduction des composés à tester dans le milieu cultivé.

Le composé n°1 possède dans ce test une dose inhibitrice de croissance ($IC_{50}$) de 25 μM alors que celle l'étrétinate est de 80 μM.

**EXEMPLE 11 : ETUDE DE LA TOXICITE SUR LES HUMERI DE FOETUS DE RATS DE 21 JOURS DE GESTATION**

La toxicité des rétinoïdes peut être évaluée sur les huméri de foetus de rats explantés in vitro d'après KISTLER (1985).

L'activité des rétinoïdes se traduit par une libération des protéoglycanes de la matrice osseuse. Cette libération est appréciée après 7 jours de culture in vitro par le dosage de la concentration de protéoglycanes dans le milieu par la méthode de WITHEMAN (1973).

Les composés de la présente invention s'avèrent totalement incapables de stimuler la libération de protéoglycanes de la matrice osseuse des huméri de foetus de rats à une concentration de 100 μM alors que l'étrétinate permet une libération significative des ces protéoglycanes à une dose de 40 μM.

**EXEMPLE 12 : DETECTION D'UNE TOXICITE DE TYPE HYPERVITAMINIQUE A IN VIVO**

Les effets secondaires limitant l'utilisation des agents rétinoÏdes en clinique humaine se traduisent par l'apparition d'un syndrome d'hypervitaminose A, celui-ci peut être reproduit expérimentalement chez l'animal.

Le protocole de traitement choisi est une injection quotidienne pendant cinq jours, reproduit deux fois, espacées de deux jours. Il est identique à celui utilisé par BOLLAG et Coll. (1981).

Alors que l'acide rétinoïque et l'étrétinate entrainent une perte de poids rapide, l'apparition d'une alopécie et la fragilisation des os, manifestations de leur toxicité, les composés de la présente invention se sont avérés dépourvus de toxicité de type hypervitaminique A : aucun signe pondéral statistiquement significatif, aucune fracture détectable par radiographie, aucun début d'alopécie n'ont été signalés suite au traitement par les composés de l'invention.

**Revendications**

**Revendications pour les Etats conractants suivants : AT, BE, CH, DE, FR, GB, IT, LI, LU, NL, SE**

1. Composés de formule générale :

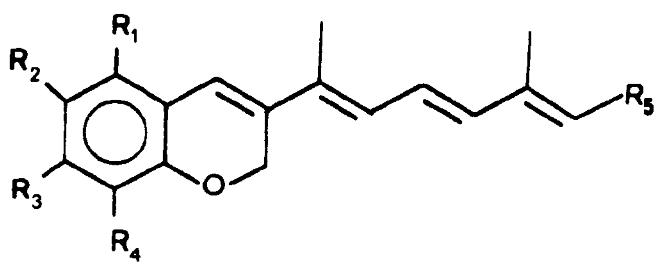

(I)

dans laquelle :

R1, R2, R3, R4 identiques ou différents représentent un atome d'hydrogène, un atome d'halogène, un groupement alcoyl inférieur, alkényl inférieur, alcoyloxy inférieur ou alkényloxy inférieur éventuellement substitués par un ou plusieurs atomes d'halogène,

$R_5$ représente un carboxyle, alcoyloxy inférieur carbonyle, alkényloxy inférieur carbonyle, alkynyloxy inférieur carbonyle,

leurs isomères, stéréoisomères, diastéréoisomères ainsi que leurs sels d'addition à une base pharmaceutiquement acceptable, lorsque R5 représente un groupement carboxyle.

2. Composé selon la revendication 1 dont la configuration est (E,E,E).

3. Composé selon l'une des revendications 1 ou 2, qui est le (diméthyl - 6,8 2H - chroményl - 3) - 7 méthyl - 3 octatriène - 2,4,6 oate d'éthyle (E,E,E).

4. Composé selon l'une des revendications 1 ou 2, qui est le (2H - chroményl - 3) - 7 méthyl - 3 octatriène

- 2,4,6 oate d'éthyle (E,E,E).

5. Composé selon l'une des revendications 1 ou 2, qui est le (chloro - 6 méthyl - 8 2H chroményl - 3) - 7 méthyl - 3 octatriène - 2,4,6 oate d'éthyle (E,E,E).

6. Composé selon l'une des revendications 1 ou 2, qui est le (2H - chroményl - 3) - 7 méthyl - 3 octatriène - 2,4,6 oate d'éthyle.

7. Procédé de préparation de composés de formule (I), selon la revendication 1 caractérisé en ce que l'on condense par chauffage à reflux du solvant un dérivé de formule (II) :

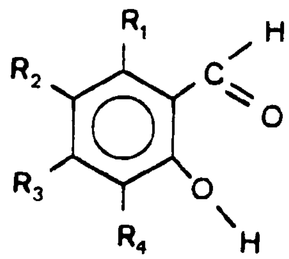

(II)

dans lequel $R_1$, $R_2$, $R_3$ et $R_4$ ont la même signification que dans la formule (I),
avec l'oxo - 3 butène - 1 de formule (III) :

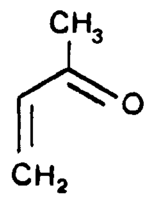

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellement choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique évaporation du solvant et purification du résidu par cristallisation ou distillation ou chromatographie sur colonne de silice ou d'alumine,

à un dérivé de formule (IV) :

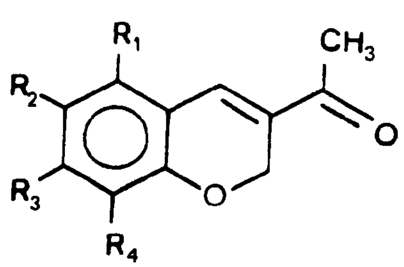

(IV)

dans laquelle $R_1$, $R_2$, $R_3$ et $R_4$ ont la même signification que dans la formule (I) lequel est soumis à hydrogénation catalytique, après dissolution dans un solvant organique convenable préférentiellement choisi parmi les alcools aliphatiques inférieurs, préférentiellement en présence d'un agent alcalin tel que l'hydroxyde de sodium, hydrogénation réalisée préférentiellement par un hydrure mixte de métal alcalin tel que le borohydrure de sodium pour conduire,

après une extraction par un solvant organique convenable tel que l'éther diéthylique, le dichlorométhane ou le chloroforme, éventuellement précédée de l'évaporation du milieu réactionnel, lavage puis évaporation de la phase organique et purification, à un dérivé de formule (V) :

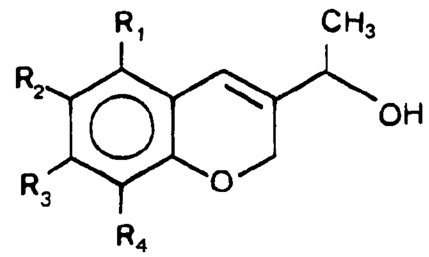

(V)

dans laquelle $R_1$, $R_2$, $R_3$ et $R_4$ ont la même signification que précédemment,

lequel est soumis à l'action d'un sel de triphényl phosphonium dans un solvant préférentiellement choisi parmi les alcools aliphatiques inférieurs à température ambiante et sous agitation pour conduire, après évaporation du solvant et purification par chromatographie sur gel de silice et cristallisation dans un solvant ou un mélange de solvants appropriés,

à un dérivé de formule (VI) :

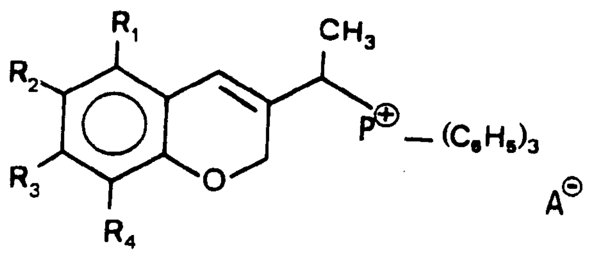

(VI)

dans lequel $A^-$ représente l'anion d'un hydracide et $R_1$, $R_2$, $R_3$ et R4 ont la même signification que précédemment,

que l'on soumet dans un solvant approprié préférentiellement, sous atmosphère inerte et en présence de n butyllithium dans l'hexane à l'action d'un aldéhyde diénique de stéréochimie bloquée sous forme d'un complexe tricarbonyl fer de formule (VII) selon la réaction décrite par WITTIG (Ber 1954, 87, 1318 et Ber 1955, 88, 1654) :

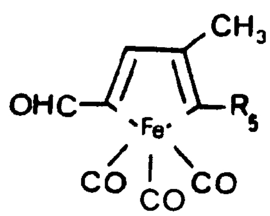

(VII)

dans lequel $R_5$ a la même signification que dans la formule (I),

à une température préférentiellement comprise entre -5°C et -30°C, préférentiellement comprise entre -15°C et -20°C, pour conduire, après extraction par un solvant organique approprié, lavage et évaporation de la phase organique à un complexe intermédiaire qui, après purification par chromatographie est soumis à reflux du solvant organique choisi pour cette étape, à l'action du N oxyde de triméthylamine,

pour conduire, après refroidissement, extraction par un solvant organique approprié, lavage et évaporation de la phase organique, et chromatographie sur colonne,

à un composé de formule (I),

que l'on peut si on le désire,

- soit lorsque $R_5$ représente un groupement carboxyle, salifier par une base pharmaceutiquement acceptable.
- soit séparer en ses stéréoisomères par une technique de cristallisation ou de chromatographie puis, si on le désire, lorsque $R_5$ représente un groupement carboxyle, salifier par une base pharmaceutiquement acceptable.

**8.** Procédé de préparation selon la revendication 7, caractérisé en ce que l'on condense par chauffage à reflux du solvant un dérivé de formule (II) :

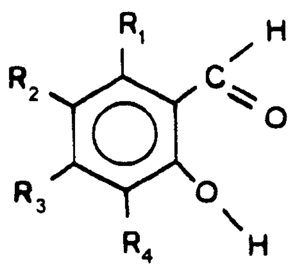

(II)

dans lequel $R_1$, $R_2$, $R_3$ et $R_4$ ont la même signification que dans la formule (I),

avec l'oxo - 3 butène - 1 de formule (III) :

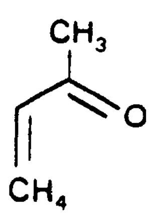

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellement choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique, évaporation du solvant et purification du résidu par cristallisation ou distillation ou chromatographie sur colonne de silice ou d'alumine,

à un dérivé de formule (IV) :

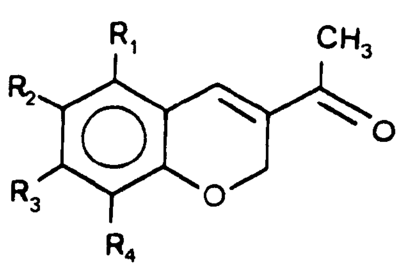

(IV)

dans laquelle $R_1$, $R_2$, $R_3$ et $R_4$ ont la même signification que dans la formule (I) lequel est soumis à l'action du bromoacétate d'éthyle en présence de zinc, suivie d'une hydrolyse extemporanée du milieu réactionnel, extraction par un solvant organique approprié préférentiellement choisi parmi acétate d'éthyle, chlorure de méthylène, chloroforme, éther diéthylique, puis à l'action de l'oxychlorure de phosphore dans un solvant organique approprié, évaporation du milieu réactionnel et nouvelle extraction par un solvant préférentiellement choisi parmi acétate d'éthyle, chlorure de méthylène, chloroforme, éther diéthylique pour conduire après une éventuelle chromatographie au gel de silice à un dérivé de formule (VIII) :

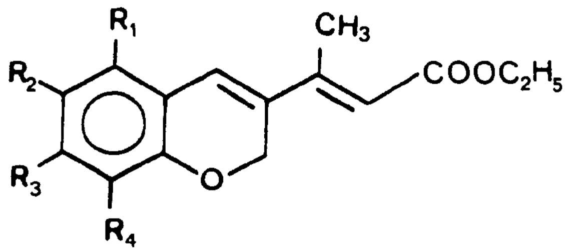

(VIII)

dans laquelle $R_1$, $R_2$, $R_3$ et $R_4$ ont la même signification que dans la formule (I),

que l'on soumet à basse température préférentiellement comprise entre - 50 et 0 °C, préférentiellement comprise entre - 25 et 5 °C dans un solvant préférentiellement choisi parmi éther diéthylique, éther diisopropylique, tétrahydrofuranne à l'action d'hydrure de lithium aluminium pour conduire à un dérivé de formule (IX) :

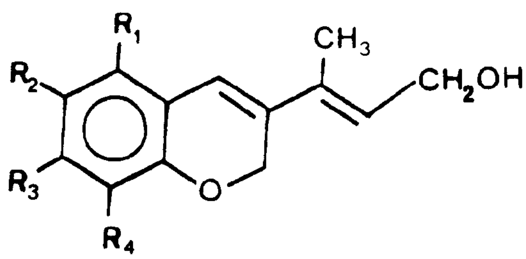

(IX)

dans laquelle $R_1$, $R_2$, $R_3$, $R_4$ ont la même signification que dans la formule (I),

que l'on soumet à oxydation par le dioxyde de manganèse pour conduire après chromatographie sur gel de silice et éventuelle cristallisation à un dérivé de formule (X) :

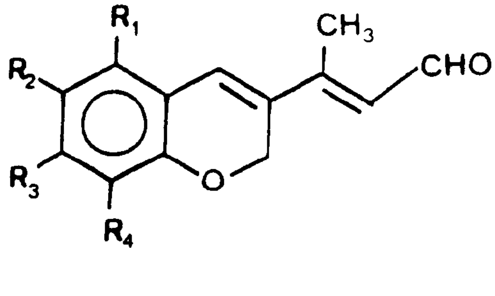

(X)

dans laquelle $R_1$, $R_2$, $R_3$ et $R_4$ ont la même signification que dans la formule (I),

que l'on soumet à l'action d'un dérivé de formule (XI) selon la réaction de WADSWORTH-EMMONS (J. Am. Chem. Soc. 1961, 83, 1733) :

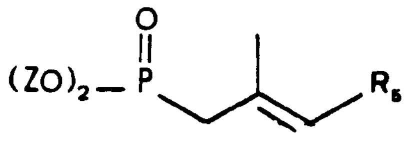

(XI)

dans laquelle $R_5$ a la même signification dans la formule (I), et Z un groupement alkyle inférieur,

à température ambiante pour conduire, après extraction par un solvant organique approprié, évaporation, purification par chromatographie sur colonne de silice à un composé de formule (I),

que l'on peut si on le désire,

- soit lorsque $R_5$ représente un groupement carboxyle, salifier par une base pharmaceutiquement acceptable.
- soit séparer en ses stéréoisomères par une technique de cristallisation ou de chromatographie puis, si on le désire, lorsque $R_5$ représente un groupement carboxyle, salifier par une base pharmaceutiquement acceptable.

**9.** Composition pharmaceutique contenant comme principe actif au moins un composé selon l'une des revendications 1 à 6 en combinaison avec un ou plusieurs excipients ou véhicules inertes, non-toxiques, pharmaceutiquement acceptables.

**10.** Composition pharmaceutique selon la revendication 9 contenant au moins un principe actif selon l'une des revendications 1 à 6 utilisables dans le traitement des tumeurs, des néoplasmes ainsi que dans les troubles cutanés divers.

**Revendications pour l'Etat contractant suivant : ES**

**1.** Procédé de préparation de composés de formule générale :

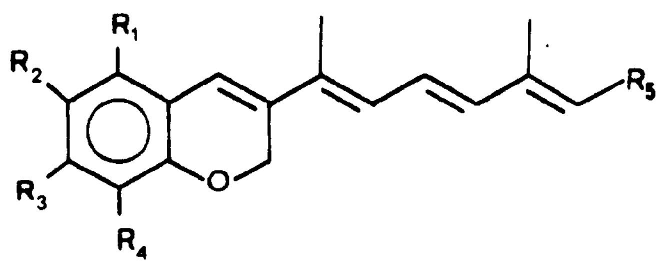

(I)

dans laquelle :

$R_1$, $R_2$, $R_3$, $R_4$ identiques ou différents représentent un atome d'hydrogène, un atome d'halogène, un groupement alcoyl inférieur, alkényl inférieur, alcoyloxy inférieur ou alkényloxy inférieur éventuellement substitués par un ou plusieurs atomes d'halogène,

$R_5$ représente un carboxyle, alcoyloxy inférieur carbonyle, alkényloxy inférieur carbonyle, alkynyloxy inférieur carbonyle,

leurs isomères, stéréoisomères, diastéréoisomères ainsi que leurs sels d'addition à une base pharmaceutiquement acceptable, lorsque R5 représente un groupement carboxyle,

caractérisé en ce que l'on condense par chauffage à reflux du solvant un dérivé de formule (II) :

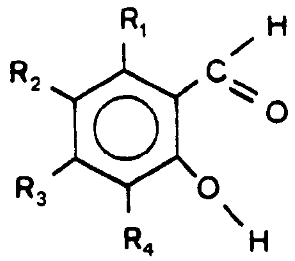

(II)

dans laquelle $R_1$, $R_2$, $R_3$ et $R_4$ ont la même signification que dans la formule (I),

avec l'oxo - 3 butène - 1 de formule (III) :

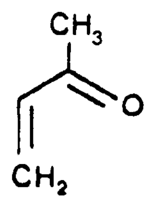

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellement choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique évaporation du solvant et purification du résidu par cristallisation ou distillation ou chromatographie sur colonne de silice ou d'alumine,

à un dérivé de formule (IV) :

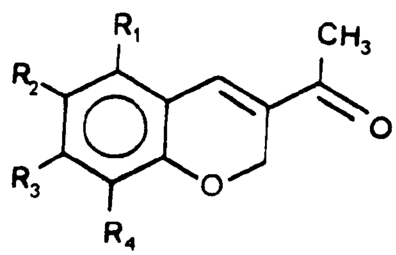

(IV)

dans laquelle $R_1$, $R_2$, $R_3$ et $R_4$ ont la même signification que dans la formule (I) lequel est soumis à hydrogénation catalytique, après dissolution dans un solvant organique convenable préférentiellement choisi parmi les alcools aliphatiques inférieurs, préférentiellement en présence d'un agent alcalin tel que l'hydroxyde de sodium, hydrogénation réalisée préférentiellement par un hydrure mixte de métal alcalin tel que le borohydrure de sodium pour conduire,

après une extraction par un solvant organique convenable tel que l'éther diéthylique, le dichlorométhane ou le chloroforme, éventuellement précédée de l'évaporation du milieu réactionnel, lavage puis évaporation de la phase organique et purification, à un dérivé de formule (V) :

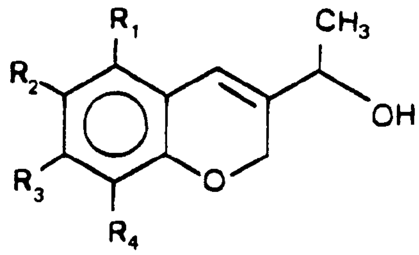

(V)

dans laquelle $R_1$, $R_2$, $R_3$ et $R_4$ ont la même signification que précédemment,

lequel est soumis à l'action d'un sel de triphényl phosphonium dans un solvant préférentiellement choisi parmi les alcools aliphatiques inférieurs à température ambiante et sous agitation pour conduire, après évaporation du solvant et purification par chromatographie sur gel de silice et cristallisation dans un solvant ou un mélange de solvants appropriés,

à un dérivé de formule (VI) :

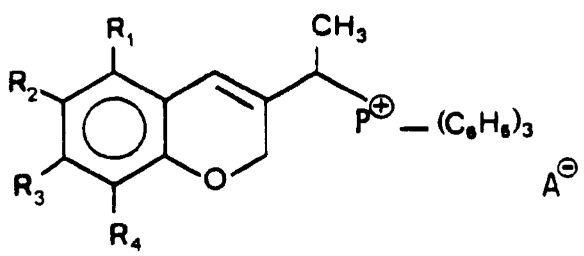

(VI)

dans lequel $A^-$ représente l'anion d'un hydracide et $R_1$, $R_2$, $R_3$ et $R_4$ ont la même signification que précédemment,

que l'on soumet dans un solvant approprié préférentiellement, sous atmosphère inerte et en présence de n butyllithium dans l'hexane à l'action d'un aldéhyde diénique de stéréochimie bloquée sous forme d'un complexe tricarbonyl fer de formule (VII) selon la réaction décrite par WITTIG (Ber 1954, 87, 1318 et Ber 1955, 88, 1654) :

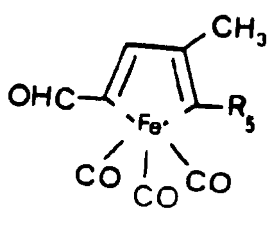

(VII)

dans lequel $R_5$ a la même signification que dans la formule (I),

à une température préférentiellement comprise entre -5°C et -30°C, préférentiellement comprise entre -15°C et -20°C, pour conduire, après extraction par un solvant organique approprié, lavage et évaporation de la phase organique à un complexe intermédiaire qui, après purification par chromatographie est soumis, à reflux du solvant organique choisi pour cette étape, à l'action du Noxyde de triméthylamine, pour conduire, après refroidissement, extraction par un solvant organique approprié, lavage et évaporation de la phase organique, et chromatographie sur colonne,

à un composé de formule (I),

que l'on peut si on le désire,

- soit lorsque $R_5$ représente un groupement carboxyle, salifier par une base pharmaceutiquement acceptable.
- soit séparer en ses stéréoisomères par une technique de cristallisation ou de chromatographie puis,

si on le désire, lorsque $R_5$ représente un groupement carboxyle, salifier par une base pharmaceutiquement acceptable.

2. Procédé de préparation selon la revendication 1, caractérisé en ce que l'on condense par chauffage à reflux du solvant un dérivé de formule (II) :

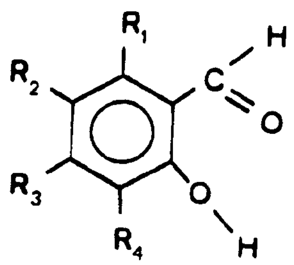

(II)

dans lequel $R_1$, $R_2$, $R_3$ et $R_4$ ont la même signification que dans la formule (I),

avec l'oxo - 3 butène - 1 de formule (III) :

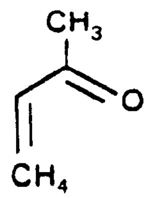

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellement choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique, évaporation du solvant et purification du résidu par cristallisation ou distillation ou chromatographie sur colonne de silice ou d'alumine,

à un dérivé de formule (IV) :

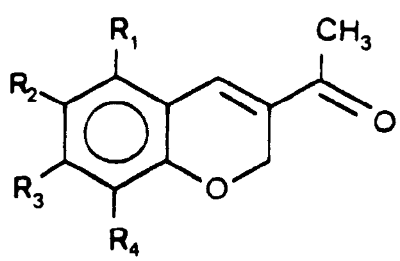

(IV)

dans laquelle $R_1$, $R_2$, $R_3$ et $R_4$ ont la même signification que dans la formule (I) lequel est soumis à l'action du bromoacétate d'éthyle en présence de zinc, suivie d'une hydrolyse extemporanée du milieu réactionnel, extraction par un solvant organique approprié préférentiellement choisi parmi acétate d'éthyle, chlorure de méthylène, chloroforme, éther diéthylique, puis à l'action de l'oxychlorure de phosphore dans un solvant organique approprié, évaporation du milieu réactionnel et nouvelle extraction par un solvant préférentiellement choisi parmi acétate d'éthyle, chlorure de méthylène, chloroforme, éther diéthylique pour conduire après une éventuelle chromatographie au gel de silice à un dérivé de formule (VIII) :

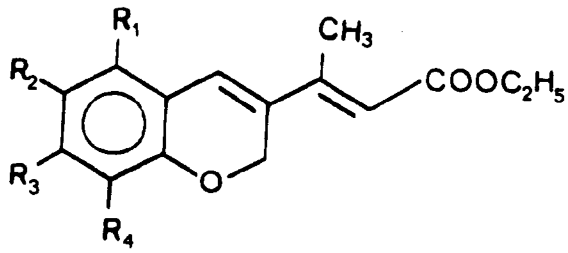

(VIII)

dans laquelle $R_1$, $R_2$, $R_3$ et $R_4$ ont la même signification que dans la formule (I),

que l'on soumet à basse température préférentiellement comprise entre - 50 et 0 °C, préférentiellement comprise entre - 25 et 5 °C dans un solvant préférentiellement choisi parmi éther diéthylique, éther diisopropylique, tétrahydrofuranne à l'action d'hydrure de lithium aluminium pour conduire à un dérivé de formule (IX) :

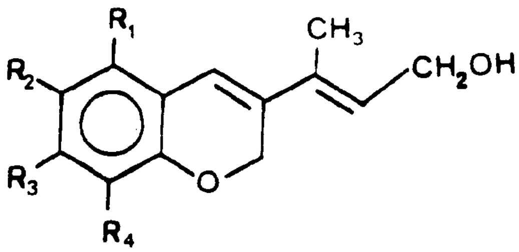

(IX)

dans laquelle $R_1$, $R_2$, $R_3$, $R_4$ ont la même signification que dans la formule (I),

que l'on soumet à oxydation par le dioxyde de manganèse pour conduire après chromatographie sur gel de silice et éventuelle cristallisation à un dérivé de formule (X) :

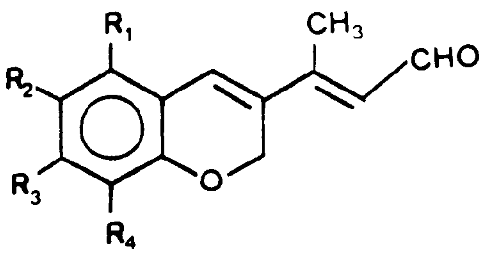

(X)

dans laquelle $R_1$, $R_2$, $R_3$, $R_4$ ont la même signification que dans la formule (I),

que l'on soumet à l'action d'un dérivé de formule (XI) selon la réaction de WADSWORTH-EMMONS (J. Am. Chem. Soc. 1961, 83, 1733) :

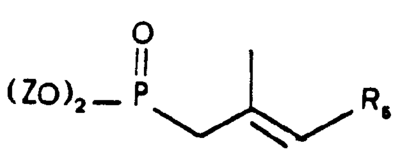

(XI)

dans laquelle $R_5$ a la même signification dans la formule (I), et Z un groupement alkyle inférieur,

à température ambiante pour conduire, après extraction par un solvant organique approprié, évaporation, purification par chromatographie sur colonne de silice à un composé de formule (I),

que l'on peut si on le désire,

- soit lorsque $R_5$ représente un groupement carboxyle, salifier par une base pharmaceutiquement acceptable.
- soit séparer en ses stéréoisomères par une technique de cristallisation ou de chromatographie puis, si on le désire, lorsque $R_5$ représente un groupement carboxyle, salifier par une base pharmaceutiquement acceptable.

3. Procédé de préparation selon la revendication 1 ou 2 de composés de formule (I) dont la configuration est (E,E,E).

4. Procédé de préparation selon l'une des revendications 1, 2 ou 3 du composé qui est le (diméthyl - 6,8 2H - chroményl - 3) - 7 méthyl - 3 octatriène - 2,4,6 oate d'éthyle (E,E,E).

5. Procédé de préparation selon l'une des revendications 1, 2 ou 3 du composé qui est le (2H - chroményl - 3) - 7 méthyl - 3 octatriène - 2,4,6 oate d'éthyle (E,E,E).

6. Procédé de préparation selon l'une des revendications 1, 2 ou 3 du composé qui est le (chloro - 6 méthyl - 8 2H chroményl - 3) - 7 méthyl - 3 octatriène - 2,4,6 oate d'éthyle (E,E,E).

7. Procédé de préparation selon l'une des revendications 1, 2 ou 3 du composé qui est le (2H - chroményl - 3) - 7 méthyl - 3 octatriène - 2,4,6 oate d'éthyle.

**Revendications pour l'Etat contractant suivant : GR**

1. Procédé de préparation de composés de formule générale :

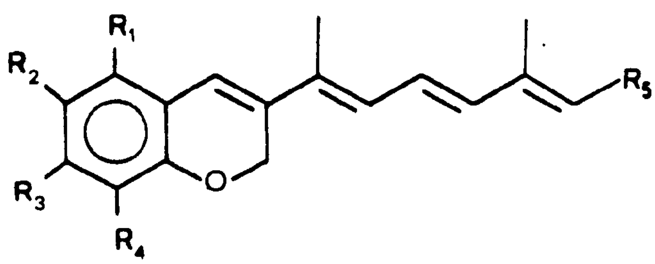

(I)

dans laquelle :

$R_1$, $R_2$, $R_3$, $R_4$ identiques ou différents représentent un atome d'hydrogène, un atome d'halogène, un groupement alcoyl inférieur, alkényl inférieur, alcoyloxy inférieur ou alkényloxy inférieur éventuellement substitués par un ou plusieurs atomes d'halogène,

$R_5$ représente un carboxyle, alcoyloxy inférieur carbonyle, alkényloxy inférieur carbonyle, alkynyloxy inférieur carbonyle,

leurs isomères, stéréoisomères, diastéréoisomères ainsi que leurs sels d'addition à une base pharmaceutiquement acceptable, lorsque R5 représente un groupement carboxyle,

caractérisé en ce que l'on condense par chauffage à reflux du solvant un dérivé de formule (II) :

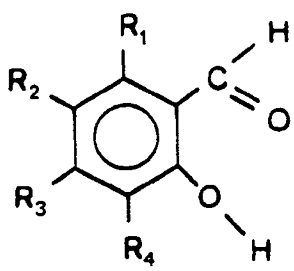

(II)

dans lequel $R_1$, $R_2$, $R_3$ et $R_4$ ont la même signification que dans la formule (I),

avec l'oxo - 3 butène - 1 de formule (III) :

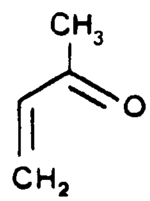

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellement choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique évaporation du solvant et purification du résidu par cristallisation ou distillation ou chromatographie sur colonne de silice ou d'alumine,

à un dérivé de formule (IV) :

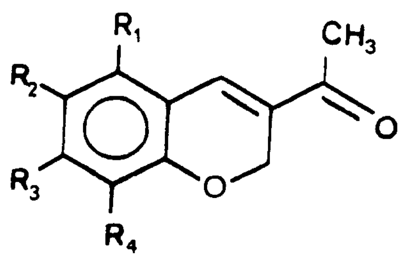

(IV)

dans laquelle $R_1$, $R_2$, $R_3$ et $R_4$ ont la même signification que dans la formule (I) lequel est soumis à hydrogénation catalytique, après dissolution dans un solvant organique convenable préférentiellement choisi parmi les alcools aliphatiques inférieurs, préférentiellement en présence d'un agent alcalin tel que l'hydroxyde de sodium, hydrogénation réalisée préférentiellement par un hydrure mixte de métal alcalin tel que le borohydrure de sodium pour conduire,

après une extraction par un solvant organique convenable tel que l'éther diéthylique, le dichlorométhane ou le chloroforme, éventuellement précédée de l'évaporation du milieu réactionnel, lavage puis évaporation de la phase organique et purification, à un dérivé de formule (V) :

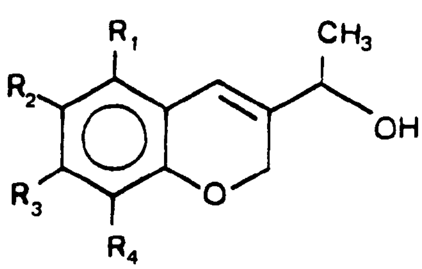

(V)

dans laquelle $R_1$, $R_2$, $R_3$ et $R_4$ ont la même signification que précédemment,

lequel est soumis à l'action d'un sel de triphényl phosphonium dans un solvant préférentiellement

choisi parmi les alcools aliphatiques inférieurs à température ambiante et sous agitation pour conduire, après évaporation du solvant et purification par chromatographie sur gel de silice et cristallisation dans un solvant ou un mélange de solvants appropriés,

à un dérivé de formule (VI) :

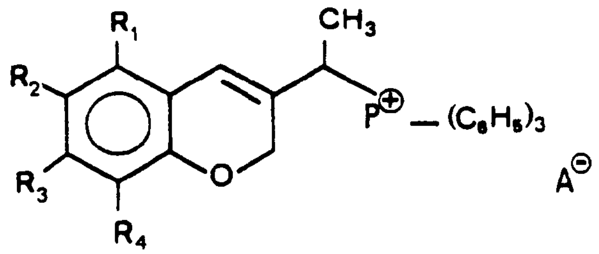

(VI)

dans lequel $A^-$ représente l'anion d'un hydracide et $R_1$, $R_2$, $R_3$ et $R_4$ ont la même signification que précédemment,

que l'on soumet dans un solvant approprié préférentiellement, sous atmosphère inerte et en présence de n butyllithium dans l'hexane à l'action d'un aldéhyde diénique de stéréochimie bloquée sous forme d'un complexe tricarbonyl fer de formule (VII) selon la réaction décrite par WITTIG (Ber 1954, 87, 1318 et Ber 1955, 88, 1654) :

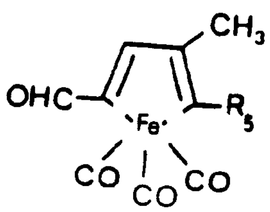

(VII)

dans lequel $R_5$ a la même signification que dans la formule (I),

à une température préférentiellement comprise entre -5°C et -30°C, préférentiellement comprise entre -15°C et -20°C, pour conduire, après extraction par un solvant organique approprié, lavage et évaporation de la phase organique à un complexe intermédiaire qui, après purification par chromatographie est soumis, à reflux du solvant organique choisi pour cette étape, à l'action du Noxyde de triméthylamine, pour conduire, après refroidissement, extraction par un solvant organique approprié, lavage et évaporation de la phase organique, et chromatographie sur colonne,

à un composé de formule (I),

que l'on peut si on le désire,

- soit lorsque $R_5$ représente un groupement carboxyle, salifier par une base pharmaceutiquement acceptable.
- soit séparer en ses stéréoisomères par une technique de cristallisation ou de chromatographie puis, si on le désire, lorsque $R_5$ représente un groupement carboxyle, salifier par une base pharmaceutiquement acceptable.

**2.** Procédé de préparation selon la revendication 1, caractérisé en ce que l'on condense par chauffage à reflux du solvant un dérivé de formule (II) :

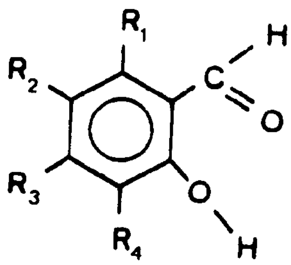

(II)

dans lequel $R_1$, $R_2$, $R_3$ et $R_4$ ont la même signification que dans la formule (I), avec l'oxo - 3 butène - 1 de formule (III) :

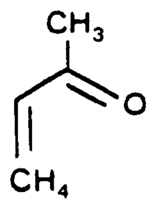

(III)

en présence d'un agent alcalin préférentiellement choisi parmi les carbonates alcalins et alcalino terreux, ou les bases organiques comme par exemple la triéthylamine ou la pyridine ou un mélange constitué d'un carbonate alcalin ou alcalino terreux et d'une base organique dans un solvant polaire préférentiellement choisi parmi les solvants cétoniques tels que la butanone - 2 ou amidiques tel que le diméthylformamide, pour conduire,

après refroidissement et éventuelle évaporation du milieu réactionnel, reprise par l'eau éventuellement additionnée d'un agent alcalin tel que l'hydroxyde de sodium, extraction par un solvant organique approprié tel que l'éther diéthylique, le chloroforme ou le chlorure de méthylène, lavage de la phase organique, évaporation du solvant et purification du résidu par cristallisation ou distillation ou chromatographie sur colonne de silice ou d'alumine,

à un dérivé de formule (IV) :

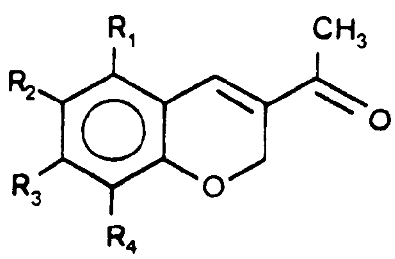

(IV)

dans laquelle $R_1$, $R_2$, $R_3$ et $R_4$ ont la même signification que dans la formule (I) lequel est soumis à l'action du bromoacétate d'éthyle en présence de zinc, suivie d'une hydrolyse extemporanée du milieu réactionnel, extraction par un solvant organique approprié préférentiellement choisi parmi acétate d'éthyle, chlorure de méthylène, chloroforme, éther diéthylique, puis à l'action de l'oxychlorure de phosphore dans un solvant organique approprié, évaporation du milieu réactionnel et nouvelle extraction par un solvant préférentiellement choisi parmi acétate d'éthyle, chlorure de méthylène, chloroforme, éther diéthylique pour conduire après une éventuelle chromatographie au gel de silice à un dérive de formule (VIII) :

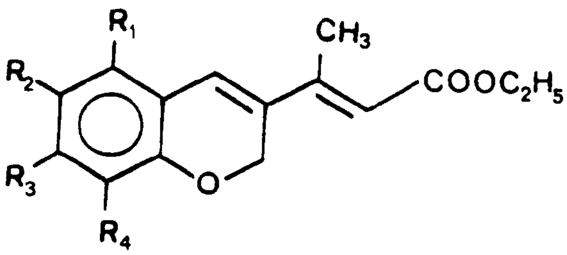

(VIII)

dans laquelle $R_1$, $R_2$, $R_3$ et $R_4$ ont la même signification que dans la formule (I),

que l'on soumet à basse température préférentiellement comprise entre - 50 et 0 °C, préférentiellement comprise entre - 25 et 5 °C dans un solvant préférentiellement choisi parmi éther diéthylique, éther diisopropylique, tétrahydrofuranne à l'action d'hydrure de lithium aluminium pour conduire à un dérivé de formule (IX) :

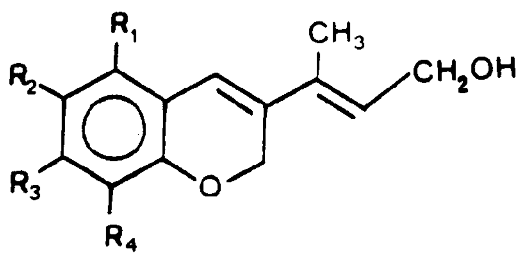

(IX)

dans laquelle $R_1$, $R_2$, $R_3$, $R_4$ ont la même signification que dans la formule (I),

que l'on soumet à oxydation par le dioxyde de manganèse pour conduire après chromatographie sur gel de silice et éventuelle cristallisation à un dérivé de formule (X) :

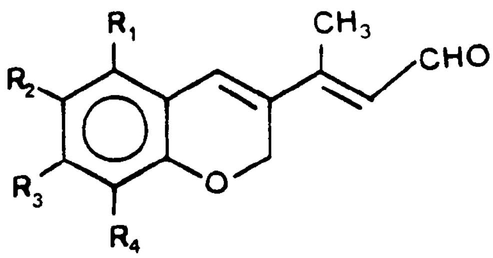

(X)

dans laquelle $R_1$, $R_2$, $R_3$, $R_4$ ont la même signification que dans la formule (I),

que l'on soumet à l'action d'un dérivé de formule (XI) selon la réaction de WADSWORTH-EMMONS (J. Am. Chem. Soc. 1961, 83, 1733) :

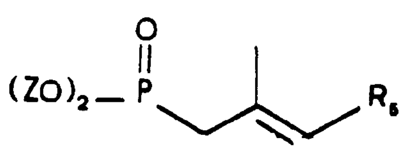

(XI)

dans laquelle $R_5$ a la même signification dans la formule (I), et Z un groupement alkyle inférieur,

à température ambiante pour conduire, après extraction par un solvant organique approprié, évaporation, purification par chromatographie sur colonne de silice à un composé de formule (I),

que l'on peut si on le désire,

- soit lorsque $R_5$ représente un groupement carboxyle, salifier par une base pharmaceutiquement acceptable.
- soit séparer en ses stéréoisomères par une technique de cristallisation ou de chromatographie puis, si on le désire, lorsque $R_5$ représente un groupement carboxyle, salifier par une base pharmaceutiquement acceptable.

3. Procédé de préparation selon la revendication 1 ou 2 de composés de formule (I) dont la configuration est (E,E,E).

4. Procédé de préparation selon l'une des revendications 1, 2 ou 3 du composé qui est le (diméthyl - 6,8 2H - chroményl - 3) - 7 méthyl - 3 octatriène - 2,4,6 oate d'éthyle (E,E,E).

5. Procédé de préparation selon l'une des revendications 1, 2 ou 3 du composé qui est le (2H - chroményl - 3) - 7 méthyl - 3 octatriène - 2,4,6 oate d'éthyle (E,E,E).

6. Procédé de préparation selon l'une des revendications 1, 2 ou 3 du composé qui est le (chloro - 6 méthyl - 8 2H chroményl - 3) - 7 méthyl - 3 octatriène - 2,4,6 oate d'éthyle (E,E,E).

7. Procédé de préparation selon l'une des revendications 1, 2 ou 3 du composé qui est le (2H - chroményl - 3) - 7 méthyl - 3 octatriène - 2,4,6 oate d'éthyle.

**Patentansprüche**

**Patentansprüche für folgende Vertragsstaaten : AT, BE, CH, DE, FR, GB, IT, LI, LU, NL, SE**

1. Verbindungen der allgemeinen Formel (I)

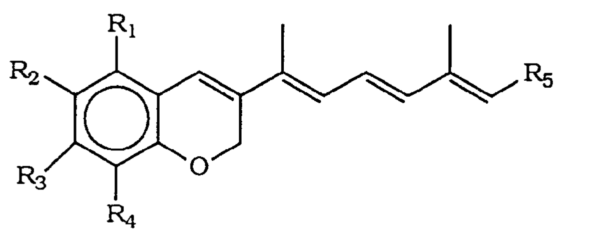

(I)

in der $R_1$, $R_2$, $R_3$ und $R_4$, die gleichartig oder verschieden sein können, Wasserstoffatome, Halogenatome, Niedrigalkylgruppen, Niedrigalkenylgruppen, Niedrigalkyloxygruppen oder Niedrigalkenyloxygruppen, die gegebenenfalls durch eines oder mehrere Halogenatome substituiert sein können, und
$R_5$ eine Carboxylgruppe, Niedrigalkyloxycarbonylgruppe, Niedrigalkenyloxycarbonylgruppe oder Niedrigalkinyloxycarbonylgruppe bedeuten, und deren Isomeren, Stereoisomeren, Diastereoisomeren und Additionssalze mit einer pharmazeutisch annehmbaren Base, wenn $R_5$ eine Carboxylgruppe darstellt.

2. Verbindung nach Anspruch 1, in der (E,E,E)-Konfiguration.

3. Verbindung nach einem der Ansprüche 1 oder 2, nämlich (E,E,E)-7-(6, 8-Dimethyl-2H-chromen-3-yl) -3-methyl-2,4,6-octatriensäureethylester.

4. Verbindung nach einem der Ansprüche 1 oder 2, nämlich (E,E,E)-7-(2H-Chromen-3-yl)-3-methyl-2,4,6-octatriensäureethylester.

5. Verbindung nach einem der Ansprüche 1 oder 2, nämlich (E,E,E)-7-(6-Chlor- 8-methyl-2 H-chromen-3-yl) -3-methyl-2,4,6-octatriensäureethylester.

6. Verbindung nach einem der Ansprüche 1 oder 2, namlich 7-(2H-Chromen-3-yl)-3-methyl-2,4,6-octatriensäureethylester.

7. Verfahren zur Herstellung der Verbindungen der Formel (I) nach Anspruch 1, **dadurch gekennzeichnet**, daß man ein Derivat der Formel (II)

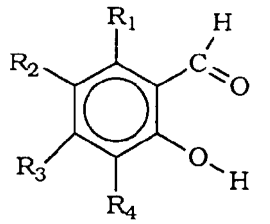

(II)

in der $R_1$, $R_2$, $R_3$ und $R_4$ die bezüglich der Formel (I) angegebenen Bedeutungen besitzen, mit 3-Oxo-1-buten der Formel (III)

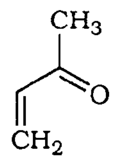

(III)

in Gegenwart eines alkalischen Mittels, welches vorzugsweise aus Alkalimetall- und Erdalkalimetall-carbonaten oder organischen Basen, wie beispielsweise Triethylamin oder Pyridin, oder einer Mischung aus einem Alkalimetall- oder Erdalkalimetall-carbonat und einer organischen Base ausgewählt ist, in einem polaren Lösungsmittel, welches vorzugsweise aus Ketonlösungsmitteln, wie Butanon-2, oder Amidlösungsmitteln, wie Dimethylformamid, ausgewählt ist, durch Erhitzen auf die Rückflußtemperatur des Lösungsmittels kondensiert, so daß man nach dem Abkühlen und eventuellem Eindampfen des Reaktionsmediums, Aufnehmen mit Wasser, dem man gegebenenfalls ein alkalisches Mittel, wie Natriumhydroxid, zugegeben hat, Extraktion mit einem geeigneten organischen Lösungsmittel, wie Diethylether, Chloroform oder Methylenchlorid, Waschen der nach dem Verdampfen des Lösungsmittels verbleibenden organischen Phase und Reinigung des Rückstands durch Kristallisation oder Destillation oder Säulenchromatographie über Siliciumdioxid oder Aluminiumoxid ein Derivat der Formel (IV) erhält,

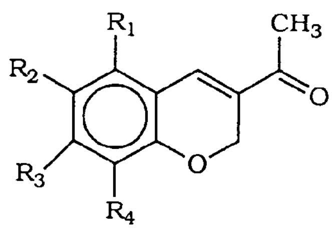

(IV)

in der $R_1$, $R_2$, $R_3$ und $R_4$ die bezüglich der Formel (I) angegebenen Bedeutungen besitzt,
welches man nach dem Auflösen in einem geeigneten organischen Lösungsmittel, welches vorzugsweise aus niedrigmolekularen aliphatischen Alkoholen ausgewählt ist, vorzugsweise in Gegenwart eines alkalischen Mittels, wie Natriumhydroxid, einer katalytischen Hydrierung unterwirft, die vorzugsweise mit einem gemischten Alkalimetallhydrid, wie Natriumborhydrid, durchgeführt wird, so daß man
nach der Extraktion mit einem geeigneten organischen Lösungsmittel, wie Diethylether, Dichlormethan oder Chloroform, die gegebenenfalls nach dem Eindampfen des Reaktionsmediums durchgeführt wird, dem Waschen und dem Verdampfen der organischen Phase und der Reinigung ein Derivat der Formel (V) erhält:

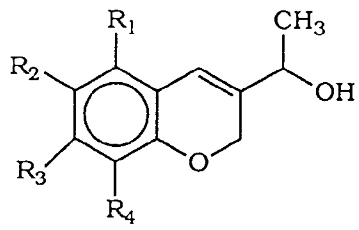

(V)

in der $R_1$, $R_2$, $R_3$ und $R_4$ die oben angegebenen Bedeutungen besitzen, welches man in einem Lösungsmittel, das vorzugsweise aus niedrigmolekularen aliphatischen Alkoholen ausgewählt ist, bei Raumtemperatur und unter Rühren mit einem Triphenylphosphoniumsalz umsetzt, so daß man nach dem Verdampfen des Lösungsmittels und der Reinigung durch Chromatographie über Kieselgel und Kristallisation in einem geeigneten Lösungsmittel oder einer geeigneten Lösungsmittelmischung,
ein Derivat der Formel (VI) erhält:

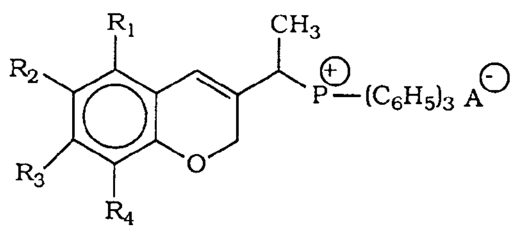

(VI)

in der $A^{\ominus}$ das Anion einer Wasserstoffsäure darstellt und $R_1$, $R_2$, $R_3$ und $R_4$ die oben angegebenen Bedeutungen besitzen,
welches man in einem geeigneten Lösungsmittel, vorzugsweise unter einer inerten Atmosphäre und in Gegenwart von n-Butyllithium in Hexan mit einem stereochemisch blockierten Dienaldehyd in Form eines Eisentricarbonylkomplexes der Formel (VII)

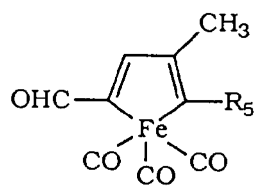

(VII)

in der
$R_5$ die bezüglich der Formel (I) angegebenen Bedeutungen besitzt, nach der von Wittig beschriebenen Reaktion (Ber. 1954, 87, 1318 und Ber. 1955, 88, 1654),
bei einer Temperatur vorzugsweise zwischen -5°C und -30°C und noch bevorzugter zwischen - 15°C und -20°C umsetzt, so daß man nach der Extraktion mit einem geeigneten organischen Lösungsmittel, dem Waschen und dem Eindampfen der organischen Phase einen Zwischenproduktkomplex erhält, der nach der Reinigung durch Chromatographie bei der Rückflußtemperatur des für diese Stufe ausgewählten organischen Lösungsmittels mit Trimethylamin-N-oxid umgesetzt wird, so daß man nach dem Abkühlen, der Extraktion mit einem geeigneten organischen Lösungsmittel, dem Waschen und dem Eindampfen der organischen Phase und der Säulenchromatographie
eine Verbindung der Formel (I) erhält,
die man gewünschtenfalls

- entweder dann, wenn $R_5$ eine Carboxylgruppe darstellt, mit einer pharmazeutisch annehmbaren Base in ein Salz überführen, oder
- durch Kristallisation oder Chromatographie in die Stereoisomeren auftrennen und dann gewünschtenfalls, wenn $R_5$ eine Carboxylgruppe darstellt, mit einer pharmazeutisch annehmbaren Base in ein Salz überführen kann.

8. Verfahren nach Anspruch 7, **dadurch gekennzeichnet**, daß man ein Derivat der Formel (II)

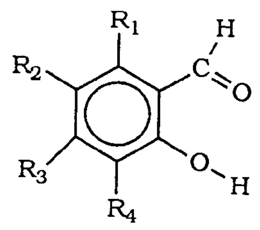

(II)

in der $R_1$, $R_2$, $R_3$ und $R_4$ die bezüglich der Formel (I) angegebenen Bedeutungen besitzen, mit 3-Oxo-1-buten der Formel (III)

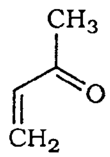

(III)

in Gegenwart eines alkalischen Mittels, welches vorzugsweise aus Alkalimetall- und Erdalkalimetall-carbonaten oder organischen Basen, wie beispielsweise Triethylamin oder Pyridin, oder einer Mischung aus einem Alkalimetall - oder Erdalkalimetall-carbonat und einer organischen Base ausgewählt ist, in einem polaren Lösungsmittel, welches vorzugsweise aus Ketonlösungsmitteln, wie Butanon-2, oder Amidlösungsmitteln, wie Dimethylformamid, ausgewählt ist, durch Erhitzen auf die Rückflußtemperatur des Lösungsmittels kondensiert,
so daß man nach dem Abkühlen und dem eventuellen Eindampfen des Reaktionsmediums, dem Aufnehmen mit Wasser, welches gegebenenfalls mit einem alkalischen Mittel, wie Natriumhydroxid, versetzt ist, der Extraktion mit einem geeigneten organischen Lösungsmittel, wie Diethylether, Chloroform oder Methylenchlorid, dem Waschen der organischen Phase, dem Verdampfen des Lösungsmittels und der Reinigung des Rückstandes durch Kristallisation oder Destillation oder Säulenchromatographie über Siliciumdioxid oder Aluminiumoxid,
ein Derivat der Formel (IV) erhält:

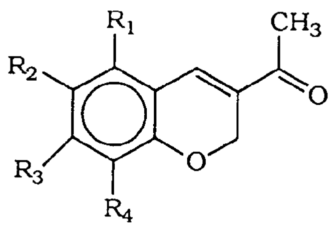

(IV)

in der $R_1$, $R_2$, $R_3$ und $R_4$ die bezüglich der Formel (I) angegebenen Bedeutungen besitzen,
welches man in Gegenwart von Zink mit Bromessigsäureethylester umsetzt, gefolgt von einer Hydrolyse des Reaktionsmediums, mit einem geeigneten organischen Lösungsmittel, welches vorzugsweise aus Ethylacetat, Methylenchlorid, Chloroform und Diethylether ausgewählt ist, extrahiert, dann mit Phosphoroxidchlorid in einem geeigneten organischen Lösungsmittel umsetzt, das Reaktionsmedium eindampft und erneut mit einem Lösungsmittel, welches vorzugsweise aus Ethylacetat, Methylenchlorid, Chloroform und Diethylether ausgewählt ist, extrahiert, so daß man nach einer gegebenenfalls durchgeführten weiteren Chromatographie über Kieselgel ein Derivat der Formel (VIII) erhält:

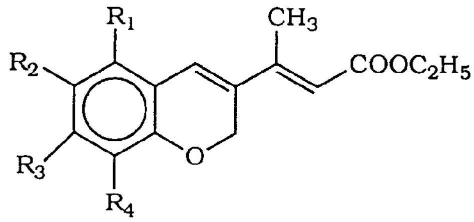

(VIII)

in der $R_1$, $R_2$, $R_3$ und $R_4$ die bezüglich der Formel (I) angegebenen Bedeutungen besitzen, welches man bei einer niedrigen Temperatur, die vorzugsweise zwischen -50 und 0°C, bevorzugter zwischen -25 und 5°C liegt, in einem Lösungsmittel, welches vorzugsweise aus Diethylether, Isopropylether und Tetrahydrofuran ausgewählt ist, mit Lithiumaluminiumhydrid umsetzt zur Bildung eines Derivats der Formel (IX)

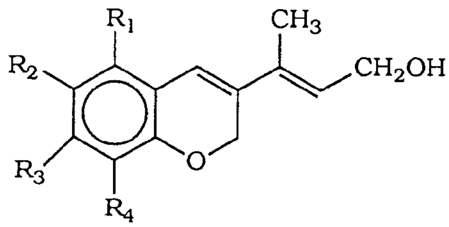

(IX)

in der $R_1$, $R_2$, $R_3$ und $R_4$ die bezüglich der Formel (I) angegebenen Bedeutungen besitzen, welches man mit Mangandioxid oxidiert, so daß man nach der Chromatographie über Kieselgel und einer gegebenenfalls durchgeführten Kristallisation ein Derivat der Formel (X) erhält:

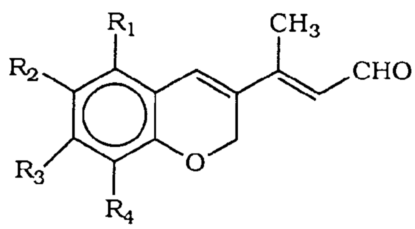

(X)

in der $R_1$, $R_2$, $R_3$ und $R_4$ die bezüglich der Formel (I) angegebenem Bedeutungen besitzen, welches man nach der Wadsworth-Emmons-Reaktion (J.Am.Chem.Soc. 1961, 83, 1733) mit einem Derivat der Formel (XI)

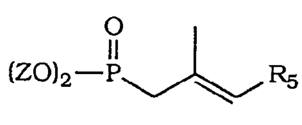

(XI)

in der $R_5$ die bezüglich der Formel (I) angegebenen Bedeutungen besitzt und Z eine Niedrigalkylgruppe darstellt, bei Raumtemperatur umsetzt, so daß man nach der Extraktion mit einem geeigneten organischen Lösungsmittel, dem Eindampfen und der Reinigung durch Säulenchromatographie über Siliciumdioxid eine Verbindung der Formel (I) erhält, die man gewünschtenfalls

- entweder dann, wenn $R_5$ eine Carboxylgruppe darstellt, mit einer pharmazeutisch annehmbaren Base in ein Salz überführen, oder
- durch Kristallisation oder Chromatographie in die Stereoisomeren auftrennen und dann gewünschtenfalls, wenn $R_5$ eine Carboxylgruppe darstellt, mit einer pharmazeutisch annehmbaren Base in ein Salz überführen kann.

**9.** Pharmazeutische Zubereitung enthaltend als Wirkstoff mindestens eine Verbindung nach einem der An-

sprüche 1 bis 6 in Kombination mit einem oder mehreren inerten, nicht-toxischen, pharmazeutisch annehmbaren Trägermaterialien oder Hilfsstoffen.

**10.** Pharmazeutische Zubereitung nach Anspruch 1 zur Behandlung von Tumoren, Neoplasmen sowie verschiedenen Hautkrankheiten enthaltend mindestens einen Wirkstoff nach einem der Ansprüche 1 bis 6.

**Patentansprüche für folgenden Vertraagsstaat : ES**

**1.** Verfahren zur Herstellung von Verbindungen der allgemeinen Formel (I)

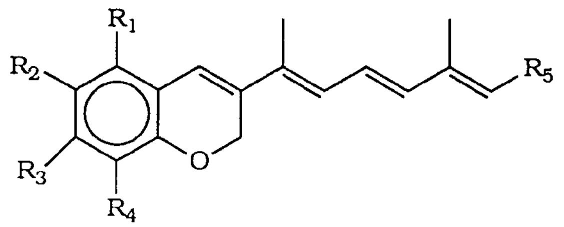

(I)

in der $R_1$, $R_2$, $R_3$ und $R_4$, die gleichartig oder verschieden sein können, Wasserstoffatome, Halogenatome, Niedrigalkylgruppen, Niedrigalkenylgruppen, Niedrigalkyloxygruppen oder Niedrigalkenyloxygruppen, die gegebenenfalls durch eines oder mehrere Halogenatome substituiert sein können, und
$R_5$ eine Carboxylgruppe, Niedrigalkyloxycarbonylgruppe, Niedrigalkenyloxycarbonylgruppe oder Niedrigalkinyloxycarbonylgruppe bedeuten, und deren Isomeren, Stereoisomeren, Diastereoisomeren und Additionssalze mit einer pharmazeutisch annehmbaren Base, wenn $R_5$ eine Carboxylgruppe darstellt, **dadurch gekennzeichnet,** daß man ein Derivat der Formel (II)

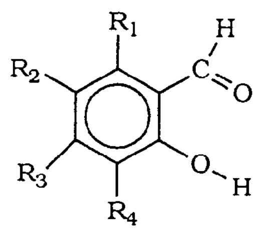

(II)

in der $R_1$, $R_2$, $R_3$ und $R_4$ die bezüglich der Formel (I) angegebenen Bedeutungen besitzen,
mit 3-Oxo-1-buten der Formel (III)

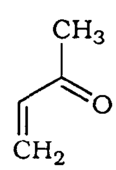

(III)

in Gegenwart eines alkalischen Mittels, welches vorzugsweise aus Alkalimetall- und Erdalkalimetall-carbonaten oder organischen Basen, wie beispielsweise Triethylamin oder Pyridin, oder einer Mischung aus einem Alkalimetall - oder Erdalkalimetall-carbonat und einer organischen Base ausgewählt ist, in einem polaren Lösungsmittel, welches vorzugsweise aus Ketonlösungsmitteln, wie Butanon-2, oder Amidlösungsmitteln, wie Dimethylformamid, ausgewählt ist, durch Erhitzen auf die Rückflußtemperatur des Lösungsmittels kondensiert, so daß man nach dem Abkühlen und eventuellem Eindampfen des Reaktionsmediums, Aufnehmen mit Wasser, dem man gegebenenfalls ein alkalisches Mittel, wie Natriumhydroxid, zugegeben hat, Extraktion mit einem geeigneten organischen Lösungsmittel, wie Diethylether, Chloroform oder Methylenchlorid, Waschen der nach dem Verdampfen des Lösungsmittels verbleibenden organischen Phase und Reinigung des Rückstands durch Kristallisation oder Destillation oder Säulenchromatographie über Siliciumdioxid oder Aluminiumoxid ein Derivat der Formel (IV) erhält:

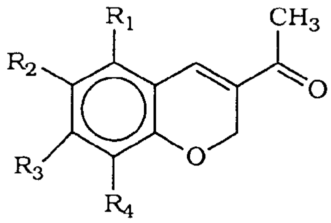

(IV)

in der $R_1$, $R_2$, $R_3$ und $R_4$ die bezüglich der Formel (I) angegebenen Bedeutungen besitzt, welches man nach dem Auflösen in einem geeigneten organischen Lösungsmittel, welches vorzugsweise aus niedrigmolekularen aliphatischen Alkoholen ausgewählt ist, vorzugsweise in Gegenwart eines alkalischen Mittels, wie Natriumhydroxid, einer katalytischen Hydrierung unterwirft, die vorzugsweise mit einem gemischten Alkalimetallhydrid, wie Natriumborhydrid, durchgeführt wird, so daß man nach der Extraktion mit einem geeigneten organischen Lösungsmittel, wie Diethylether, Dichlormethan oder Chloroform, die gegebenenfalls nach dem Eindampfen des Reaktionsmediums durchgeführt wird, dem Waschen und dem Verdampfen der organischen Phase und der Reinigung ein Derivat der Formel (V) erhält:

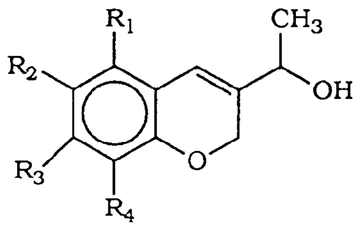

(V)

in der $R_1$, $R_2$, $R_3$ und $R_4$ die oben angegebenen Bedeutungen besitzen, welches man in einem Lösungsmittel, das vorzugsweise aus niedrigmolekularen aliphatischen Alkoholen ausgewählt ist, bei Raumtemperatur und unter Rühren mit einem Triphenylphosphoniumsalz umsetzt, so daß man nach dem Verdampfen des Lösungsmittels und der Reinigung durch Chromatographie über Kieselgel und Kristallisation in einem geeigneten Lösungsmittel oder einer geeigneten Lösungsmittelmischung,
ein Derivat der Formel (VI)

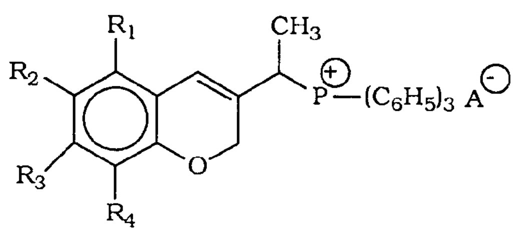

(VI)

in der $A^{\ominus}$ das Anion einer Wasserstoffsäure darstellt und $R_1$, $R_2$, $R_3$ und $R_4$ die oben angegebenen Bedeutungen besitzen,
welches man in einem geeigneten Lösungsmittel, vorzugsweise unter ein-erinerten Atmosphäre und in Gegenwart von n-Butyllithium in Hexan mit einem stereochemisch blockierten Dienaldehyd in Form eines Eisentricarbonylkomplexes der Formel (VII)

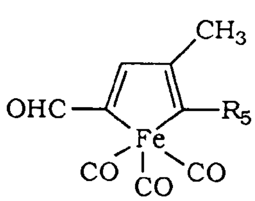

(VII)

in der
$R_5$ die bezüglich der Formel (I) angegebenen Bedeutungen besitzt, nach der von Wittig beschriebenen Reaktion (Ber. 1954, 87, 1318 und Ber. 1955, 88, 1654),
bei einer Temperatur vorzugsweise zwischen -5°C und -30°C und noch bevorzugter zwischen -15°C und -20°C umsetzt, so daß man nach der Extraktion mit einem geeigneten organischen Lösungsmittel, dem Waschen und dem Eindampfen der organischen Phase einen Zwischenproduktkomplex erhält, der nach der Reinigung durch Chromatographie bei der Rückflußtemperatur des für diese Stufe ausgewählten organischen Lösungsmittels mit Trimethylamin-N-oxid umgesetzt wird, so daß man nach dem Abkühlen, der Extraktion mit einem geeigneten organischen Lösungsmittel, dem Waschen und dem Eindampfen der organischen Phase und der Säulenchromatographie
eine Verbindung der Formel (I) erhält,
die man gewünschtenfalls

- entweder dann, wenn $R_5$ eine Carboxylgruppe darstellt, mit einer pharmazeutisch annehmbaren Base in ein Salz überführen, oder
- durch Kristallisation oder Chromatographie in die Stereoisomeren auftrennen und dann gewünschtenfalls, wenn $R_5$ eine Carboxylgruppe darstellt, mit einer pharmazeutisch annehmbaren Base in ein Salz überführen kann.

2. Verfahren nach Anspruch 1, **dadurch gekennzeichnet,** daß man ein Derivat der Formel (II)

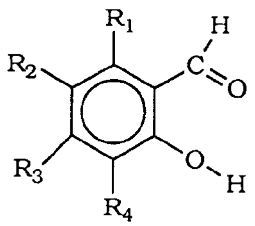

(II)

in der $R_1$, $R_2$, $R_3$ und $R_4$ die bezüglich der Formel (I) angegebenen Bedeutungen besitzen, mit 3-Oxo-1-buten der Formel (III)

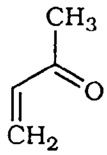

(III)

in Gegenwart eines alkalischen Mittels, welches vorzugsweise aus Alkalimetall- und Erdalkalimetall-carbonaten oder organischen Basen, wie beispielsweise Triethylamin oder Pyridin, oder einer Mischung aus einem Alkalimetall- oder Erdalkalimetall-carbonat und einer organischen Base ausgewählt ist, in einem polaren Lösungsmittel, welches vorzugsweise aus Ketonlösungsmitteln, wie Butanon-2, oder Amidlösungsmitteln, wie Dimethylformamid, durch Erhitzen auf die Rückflußtemperatur des Lösungsmittels kondensiert,
so daß man nach dem Abkühlen und dem eventuellen Eindampfen des Reaktionsmediums, dem Aufnehmen mit Wasser, welches gegebenenfalls mit einem alkalischen Mittel, wie Natriumhydroxid versetzt ist, der Extraktion mit einem geeigneten organischen Lösungsmittel, wie Diethylether, Chloroform oder Methylenchlorid, dem Waschen der organischen Phase, dem Verdampfen des Lösungsmittels und der Reinigung des Rückstandes durch Kristallisation oder Destillation oder Säulenchromatographie über Siliciumdioxid oder Aluminiumoxid,
ein Derivat der Formel (IV) erhält:

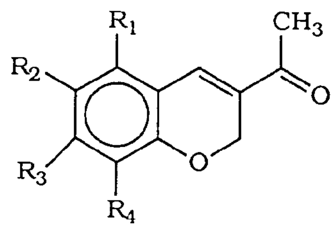

(IV)

in der $R_1$, $R_2$, $R_3$ und $R_4$ die bezüglich der Formel (I) angegebenen Bedeutungen besitzen,
welches man in Gegenwart von Zink mit Bromessigsäureethylester umsetzt, gefolgt von einer Hydrolyse des Reaktionsmediums, mit einem geeigneten organischen Lösungsmittel, welches vorzugsweise aus Ethylacetat, Methylenchlorid, Chloroform und Diethylether ausgewählt ist, extrahiert, dann mit Phosphoroxidchlorid in einem geeigneten organischen Lösungsmittel umsetzt, das Reaktionsmedium eindampft und erneut mit einem Lösungsmittel, welches vorzugsweise aus Ethylacetat, Methylenchlorid, Chloroform und Diethylether ausgewählt ist, extrahiert, so daß man nach einer gegebenenfalls durchgeführten weiteren Chromatographie über Kieselgel ein Derivat der Formel (VIII) erhält:

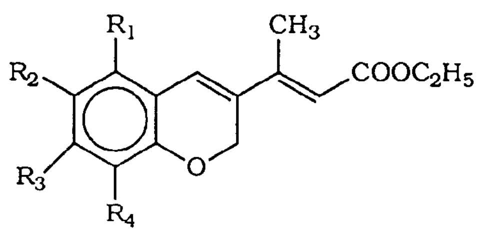

(VIII)

in der $R_1$, $R_2$, $R_3$ und $R_4$ die bezüglich der Formel (I) angegebenen Bedeutungen besitzen,
welches man bei einer niedrigen Temperatur, die vorzugsweise zwischen -50 und 0°C, bevorzugter zwischen -25 und 5°C liegt, in einem Lösungsmittel, welches vorzugsweise aus Diethylether, Isopropylether und Tetrahydrofuran ausgewählt ist, mit Lithiumaluminiumhydrid umsetzt zur Bildung eines Derivats der Formel (IX)

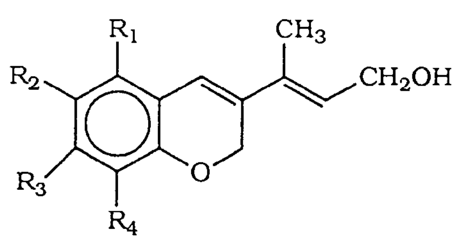

(IX)

in der $R_1$, $R_2$, $R_3$ und $R_4$ die bezüglich der Formel (I) angegebenen Bedeutungen besitzen,
welches man mit Mangandioxid oxidiert, so daß man nach der Chromatographie über Kieselgel und einer gegebenenfalls durchgeführten Kristallisation ein Derivat der Formel (X) erhält:

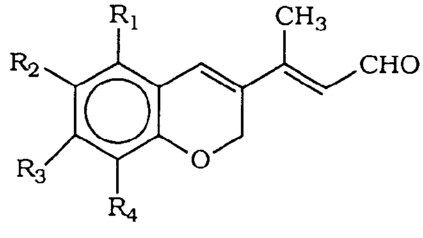

(X)

in der $R_1$, $R_2$, $R_3$ und $R_4$ die bezüglich der Formel (I) angegebenen Bedeutungen besitzen, welches man nach der Wadsworth-Emmons-Reaktion (J.Am. Chem. Soc. 1961, 83, 1733) mit einem Derivat der Formel (XI)

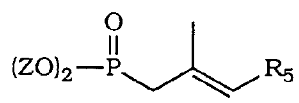

(XI)

in der $R_5$ die bezüglich der Formel (I) angegebenen Bedeutungen besitzt und Z eine Niedrigalkylgruppe darstellt, bei Raumtemperatur umsetzt, so daß man nach der Extraktion mit einem geeigneten organischen Lösungsmittel, dem Eindampfen und der Reinigung durch Säulenchromatographie über Siliciumdioxid eine Verbindung der Formel (I) erhält, die man gewünschtenfalls

- entweder dann, wenn $R_5$ eine Carboxylgruppe darstellt, mit einer pharmazeutisch annehmbaren Base in ein Salz überführen, oder
- durch Kristallisation oder Chromatographie in die Stereoisomeren auftrennen und dann gewünschtenfalls, wenn $R_5$ eine Carboxylgruppe darstellt, mit einer pharmazeutisch annehmbaren Base in ein Salz überführen kann.

3. Verfahren nach Anspruch 1 oder 2 zur Herstellung von Verbindungen der Formel (I) in der (E,E,E)-Konfiguration.

4. Verfahren nach einem der Ansprüche 1, 2 oder 3, zur Herstellung von (E,E,E)-7-(6,8-Dimethyl-2H-chromen-3-yl)-3-methyl-2,4,6-octatriensäureethylester.

5. Verfahren nach einem der Ansprüche 1, 2 oder 3 zur Herstellung von (E,E,E)-7-(2H-Chromen-3-yl)-3-methyl-2,4,6-octatriensäureethylester.

6. Verfahren nach einem der Ansprüche 1, 2 oder 3 zur Herstellung von (E,E,E)-7-(6-Chlor-8-methyl-2H-chromen-3-yl)-3-methyl-2,4,6-octatriensäureethylester.

7. Verfahren nach einem der Ansprüche 1, 2 oder 3 zur Herstellung von 7-(2H-Chromen-3-yl)-3-methyl-2,4,6-octatriensäureethylester.

**Patentansprüche für folgenden Vertraagsstaat : GR**

1. Verfahren zur Herstellung von Verbindungen der allgemeinen Formel (I)

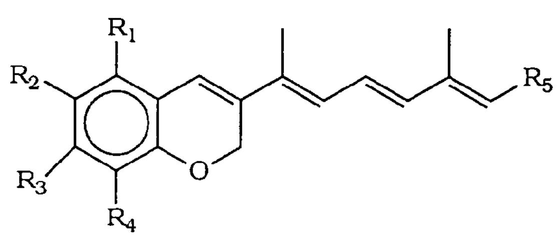

(I)

in der $R_1$, $R_2$, $R_3$ und $R_4$, die gleichartig oder verschieden sein können, Wasserstoffatome, Halogenatome, Niedrigalkylgruppen, Niedrigalkenylgruppen, Niedrigalkyloxygruppen oder Niedrigalkenyloxygruppen, die gegebenenfalls durch eines oder mehrere Halogenatome substituiert sein können, und $R_5$ eine Carboxylgruppe, Niedrigalkyloxycarbonylgruppe, Niedrigalkenyloxycarbonylgruppe oder Niedrigalkinyloxycarbonylgruppe bedeuten, deren Isomeren, Stereoisomeren, Diastereoisomeren und Additionssalze mit einer pharmazeutisch annehmbaren Base, wenn $R_5$ eine Carboxylgruppe darstellt, **dadurch gekennzeichnet**, daß man ein Derivat der Formel (II)

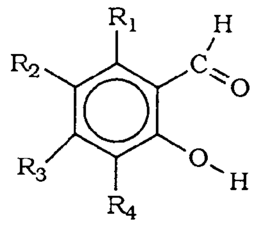

(II)

in der $R_1$, $R_2$, $R_3$ und $R_4$ die bezüglich der Formel (I) angegebenen Bedeutungen besitzen, mit 3-Oxo-1-buten der Formel (III)

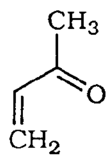

(III)

in Gegenwart eines alkalischen Mittels, welches vorzugsweise aus Alkalimetall- und Erdalkalimetall-carbonaten oder organischen Basen, wie beispielsweise Triethylamin oder Pyridin, oder einer Mischung aus einem Alkalimetall - oder Erdalkalimetall-carbonat und einer organischen Base ausgewählt ist, in einem polaren Lösungsmittel, welches vorzugsweise aus Ketonlösungsmitteln, wie Butanon-2, oder Amidlösungsmitteln, wie Dimethylformamid, ausgewählt ist, durch Erhitzen auf die Rückflußtemperatur des Lösungsmittels kondensiert, so daß man nach dem Abkühlen und eventuellem Eindampfen des Reaktionsmediums, Aufnehmen mit Wasser, dem man gegebenenfalls ein alkalisches Mittel, wie Natriumhydroxid, zugegeben hat, Extraktion mit einem geeigneten organischen Lösungsmittel, wie Diethylether, Chloroform oder Methylenchlorid, Waschen der nach dem Verdampfen des Lösungsmittels verbleibenden organischen Phase und Reinigung des Rückstands durch Kristallisation oder Destillation oder Säulenchromatographie über Siliciumdioxid oder Aluminiumoxid ein Derivat der Formel (IV) erhält:

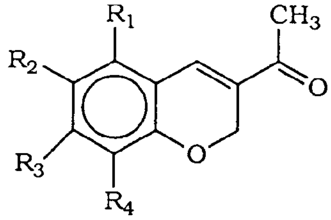

(IV)

in der $R_1$, $R_2$, $R_3$ und $R_4$ die bezüglich der Formel (I) angegebenen Bedeutungen besitzt,
welches man nach dem Auflösen in einem geeigneten organischen Lösungsmittel, welches vorzugsweise aus niedrigmolekularen aliphatischen Alkoholen ausgewählt ist, vorzugsweise in Gegenwart eines alkalischen Mittels, wie Natriumhydroxid, einer katalytischen Hydrierung unterwirft, die vorzugsweise mit einem gemischten Alkalimetallhydrid, wie Natriumborhydrid, durchgeführt wird, so daß man
nach der Extraktion mit einem geeigneten organischen Lösungsmittel, wie Diethylether, Dichlormethan oder Chloroform, die gegebenenfalls nach dem Eindampfen des Reaktionsmediums durchgeführt wird, dem Waschen und dem Verdampfen der organischen Phase und der Reinigung ein Derivat der Formel (V) erhält:

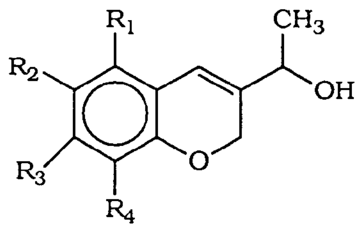

(V)

in der $R_1$, $R_2$, $R_3$ und $R_4$ die oben angegebenen Bedeutungen besitzen, welches man in einem Lösungsmittel, das vorzugsweise aus niedrigmolekularen aliphatischen Alkoholen ausgewählt ist, bei Raumtempera- tur und unter Rühren mit einem Triphenylphosphoniumsalz umsetzt, so daß man nach dem Verdampfen des Lösungsmittels und der Reinigung durch Chromatographie über Kieselgel und Kristallisation in einem geeigneten Lösungsmittel oder einer geeigneten Lösungsmittelmischung, ein Derivat der Formel (VI) erhält:

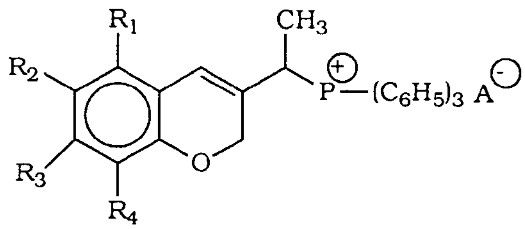

(VI)

in der $A^{\ominus}$ das Anion einer Wasserstoffsäure darstellt und $R_1$, $R_2$, $R_3$ und $R_4$ die oben angegebenen Bedeutungen besitzen,
welches man in einem geeigneten Lösungsmittel, vorzugsweise unter einer inerten Atmosphäre und in Gegenwart von n-Butyllithium in Hexan mit einem stereochemisch blockierten Dienaldehyd in Form eines Eisentricarbonylkomplexes der Formel (VII)

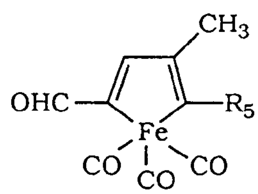

(VII)

in der
$R_5$ die bezüglich der Formel (I) angegebenen Bedeutungen besitzt, nach der von Wittig beschriebenen Reaktion (Ber. 1954, 87, 1318 und Ber. 1955, 88, 1654),
bei einer Temperatur vorzugsweise zwischen -5°C und -30°C und noch bevorzugter zwischen -15°C und -20°C umsetzt, so daß man nach der Extrak.tion mit einem geeigneten organischen Lösungsmittel, dem Waschen und dem Eindampfen der organischen Phase einen Zwischenproduktkomplex erhält, der nach der Reinigung durch Chromatographie bei der Rückflußtemperatur des für diese Stufe ausgewählten organischen Lösungsmittels mit Trimethylamin-N-oxid umgesetzt wird, so daß man nach dem Abkühlen, der Extraktion mit einem geeigneten organischen Lösungsmittel, dem Waschen und dem Eindampfen der organischen Phase und der Säulenchromatographie
eine Verbindung der Formel (I) erhält,
die man gewünschtenfalls

- entweder dann, wenn $R_5$ eine Carboxylgruppe darstellt, mit einer pharmazeutisch annehmbaren Base in ein Salz überführen, oder
- durch Kristallisation oder Chromatographie in die Stereoisomeren auftrennen und dann gewünschtenfalls, wenn $R_5$ eine Carboxylgruppe darstellt, mit einer pharmazeutisch annehmbaren Base in ein Salz überführen kann.

2. Verfahren nach Anspruch 1, **dadurch gekennzeichnet,** daß man ein Derivat der Formel (II)

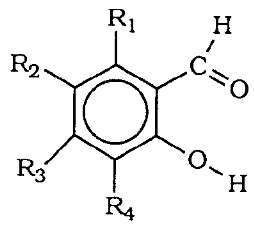

(II)

in der $R_1$, $R_2$, $R_3$ und $R_4$ die bezüglich der Formel (I) angegebenen Bedeutungen besitzen, mit 3-Oxo-1-buten der Formel (III)

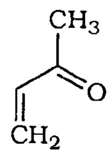

(III)

in Gegenwart eines alkalischen Mittels, welches vorzugsweise aus Alkalimetall- und Erdalkalimetall-carbonaten oder organischen Basen, wie beispielsweise Triethylamin oder Pyridin, oder einer Mischung aus einem Alkalimetall- oder Erdalkalimetall-carbonat und einer organischen Base ausgewählt ist, in einem polaren Lösungsmittel, welches vorzugsweise aus Ketonlösungsmitteln, wie Butanon-2, oder Amidlösungsmitteln, wie Dimethylformamid, ausgewählt ist, durch Erhitzen auf die Rückflußtemperatur des Lösungsmittels kondensiert,
so daß man nach dem Abkühlen und dem eventuellen Eindampfen des Reaktionsmediums, dem Aufnehmen mit Wasser, welches gegebenenfalls mit einem alkalischen Mittel, wie Natriumhydroxid, versetzt ist, der Extraktion mit einem geeigneten organischen Lösungsmittel, wie Diethylether, Chloroform oder Methylenchlorid, dem Waschen der organischen Phase, dem Verdampfen des Lösungsmittels und der Reinigung des Rückstandes durch Kristallisation oder Destillation oder Säulenchromatographie über Siliciumdioxid oder Aluminiumoxid,
ein Derivat der Formel (IV) erhält:

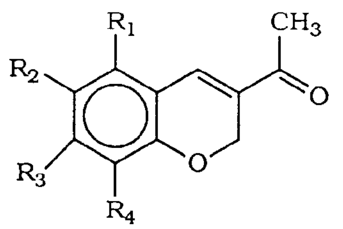

(IV)

in der $R_1$, $R_2$, $R_3$ und $R_4$ die bezüglich der Formel (I) angegebenen Bedeutungen besitzen,
welches man in Gegenwart von Zink mit Bromessigsäureethylester umsetzt, gefolgt von einer Hydrolyse des Reaktionsmediums, mit einem geeigneten organischen Lösungsmittel, welches vorzugsweise aus Ethylacetat, Methylenchlorid, Chloroform und Diethylether ausgewählt ist, extrahiert, dann mit Phosphoroxidchlorid in einem geeigneten organischen Lösungsmittel umsetzt, das Reaktionsmedium eindampft und erneut mit einem Lösungsmittel, welches vorzugsweise aus Ethylacetat, Methylenchlorid, Chloroform und Diethylether ausgewählt ist, extrahiert, so daß man nach einer gegebenenfalls durchgeführten weiteren Chromatographie über Kieselgel ein Derivat der Formel (VIII) erhält:

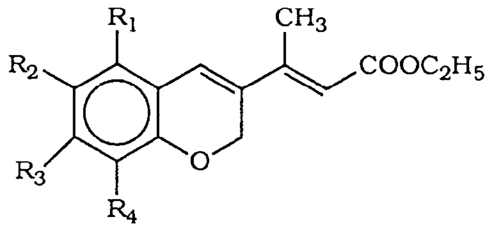

(VIII)

in der $R_1$, $R_2$, $R_3$ und $R_4$ die bezüglich der Formel (I) angegebenen Bedeutungen besitzen, welches man bei einer niedrigen Temperatur, die vorzugsweise zwischen -50 und 0°C, bevorzugter zwischen -25 und 5°C liegt, in einem Lösungsmittel, welches vorzugsweise aus Diethylether, Isopropylether und Tetrahydrofuran ausgewählt ist, mit Lithiumaluminiumhydrid umsetzt zur Bildung eines Derivats der Formel (IX)

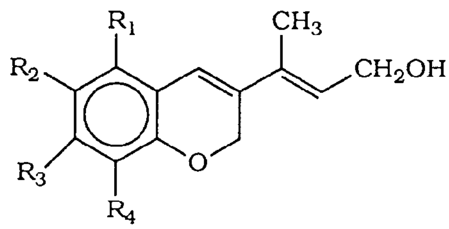

(IX)

in der $R_1$, $R_2$, $R_3$ und $R_4$ die bezüglich der Formel (I) angegebenen Bedeutungen besitzen, welches man mit Mangandioxid oxidiert, so daß man nach der Chromatographie über Kieselgel und einer gegebenenfalls durchgeführten Kristallisation ein Derivat der Formel (X) erhält:

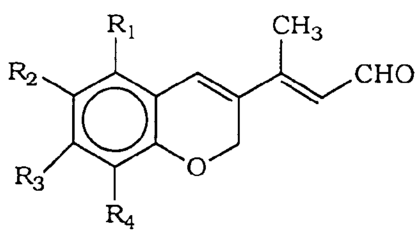

(X)

in der $R_1$, $R_2$, $R_3$ und $R_4$ die bezüglich der Formel (I) angegebenen Bedeutungen besitzen, welches man nach der Wadsworth-Emmons-Reaktion (J.Am.Chem.Soc. 1961, 83, 1733) mit einem Derivat der Formel (XI)

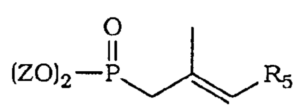

(XI)

in der $R_5$ die bezüglich der Formel (I) angegebenen Bedeutungen besitzt und Z eine Niedrigalkylgruppe darstellt, bei Raumtemperatur umsetzt, so daß man nach der Extraktion mit einem geeigneten organischen Lösungsmittel, dem Eindampfen und der Reinigung durch Säulenchromatographie über Siliciumdioxid eine Verbindung der Formel (I) erhält, die man gewünschtenfalls

- entweder dann, wenn $R_5$ eine Carboxylgruppe darstellt, mit einer pharmazeutisch annehmbaren Base in ein Salz überführen, oder
- durch Kristallisation oder Chromatographie in die Stereoisomeren auftrennen und dann gewünschtenfalls, wenn $R_5$ eine Carboxylgruppe darstellt, mit einer pharmazeutisch annehmbaren Base in ein Salz überführen kann.

3. Verfahren nach Anspruch 1 oder 2 zur Herstellung von Verbindungen der Formel (I) in der (E,E,E)-Konfiguration.

4. Verfahren nach einem der Ansprüche 1, 2 oder 3, zur Herstellung von (E,E,E)-7-(6,8-Dimethyl-2H-chromen-3-yl)-3-methyl-2,4,6-octatriensäureethylester.

5. Verfahren nach einem der Ansprüche 1, 2 oder 3 zur Herstellung von (E,E,E)-7-(2H-Chromen-3-yl)-3-methyl-2,4,6-octatriensäureethylester.

6. Verfahren nach einem der Ansprüche 1, 2 oder 3 zur Herstellung von (E,E,E)-7-(6-Chlor-8-methyl-2H-chromen-3-yl)-3-methyl-2,4,6-octatriensäureethylester.

7. Verfahren nach einem der Ansprüche 1, 2 oder 3 zur Herstellung von 7-(2H-Chromen-3-yl)-3-methyl-2,4,6-octatriensäureethylester.

## Claims

**Claims for the following Contracting States : AT, BE, CH, DE, FR, GB, IT, LI, LU, NL, SE**

1. Compounds of the general formula:

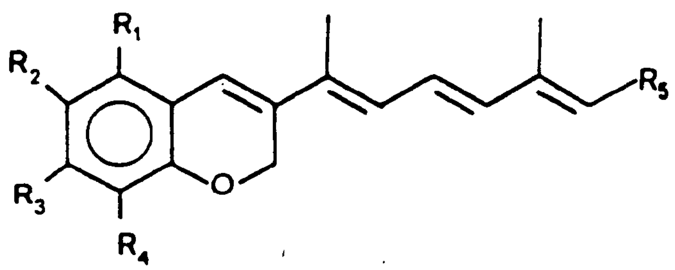

(I)

in which:

$R_1$, $R_2$, $R_3$ and $R_4$, which may be the same or different, represent a hydrogen atom, a halogen atom, a lower alkyl, lower alkenyl, lower alkoxy or lower alkenyloxy group, each of which is optionally substituted by one or more halogen atoms, and

$R_5$ represents a carboxy, lower alkoxycarbonyl, lower alkenyloxycarbonyl or lower alkynyloxycarbonyl group,

their isomers, stereoisomers, diastereoisomers and also their addition salts with a pharmaceutically acceptable base, when $R_5$ represents a carboxy group.

2. Compound according to claim 1 of which the configuration is (E,E,E).

3. Compound according to either claim 1 or claim 2, which is ethyl (E,E,E)-7-(6,8-dimethyl-2H-chromen-3-yl)-3-methyl-2,4,6-octatriene carboxylate.

4. Compound according to either claim 1 or claim 2, which is ethyl (E,E,E)-7-(2H-chromen-3-yl)-3-methyl-2,4,6-octatriene carboxylate.

5. Compound according to either claim 1 or claim 2, which is ethyl (E,E,E)-7-(6-chloro-8-methyl-2H-chromen-3-yl)-3-methyl-2,4,6-octatriene carboxylate.

6. Compound according to either claim 1 or claim 2, which is ethyl 7-(2H-chromen-3-yl)-3-methyl-2,4,6-octatriene carboxylate.

7. Process for the preparation of compounds of the formula (I) according to claim 1, characterised in that a compound of the formula (II):

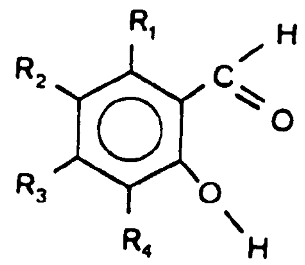

(II)

in which $R_1$, $R_2$, $R_3$ and $R_4$ are as defined in formula (I), is condensed by heating at the reflux temperature of the solvent

with 3-oxo-1-butene of the formula (III):

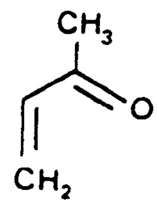

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from ketone solvents, such as 2-butanone, or amide solvents, such as dimethylformamide, to yield,

after cooling and optionally evaporating the reaction medium, taking up in water to which an alkaline agent, such as sodium hydroxide, has optionally been added, extracting with a suitable organic solvent, such as diethyl ether, chloroform or methylene chloride, washing the organic phase, evaporating the solvent and purifying the residue by crystallisation, or distillation, or chromatography on a silica or aluminium oxide column,

a compound of the formula (IV):

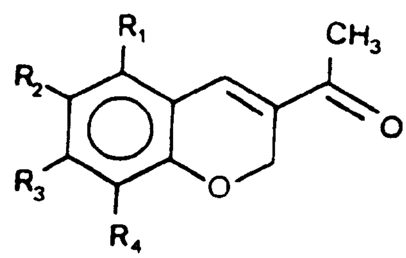

(IV)

in which $R_1$, $R_2$, $R_3$ and $R_4$ are as defined in formula (I), which, after being dissolved in a suitable organic solvent preferably selected from the lower aliphatic alcohols, is subjected, preferably in the presence of an alkaline agent, such as sodium hydroxide, to catalytic hydrogenation, preferably using a mixed alkali metal hydride, such as sodium borohydride, to yield,

after extracting with a suitable organic solvent, such as diethyl ether, dichloromethane or chloroform, optionally preceded by evaporation of the reaction medium, washing and then evaporating the organic phase and purifying, a compound of the formula (V):

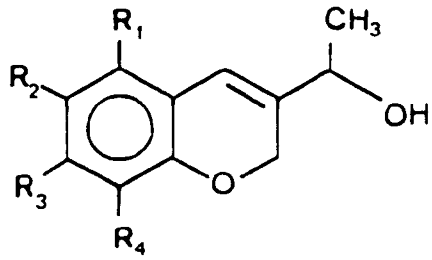

(V)

in which $R_1$, $R_2$, $R_3$ and $R_4$ are as defined above, which is subjected to the action of a triphenylphosphonium salt in a solvent preferably selected from the lower aliphatic alcohols, at ambient temperature and with stirring, to yield, after evaporating the solvent and purifying by chromatography on silica gel and crystallisation in a suitable solvent or a mixture of suitable solvents,

a compound of the formula (VI):

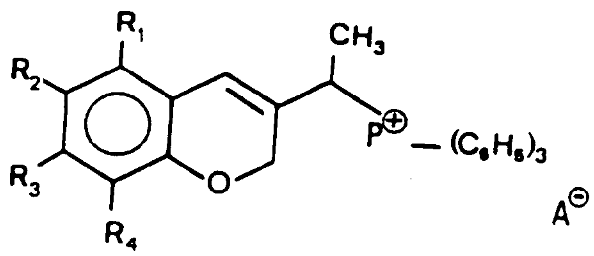

(VI)

in which $A^{\ominus}$ represents the anion of a hydracid and $R_1$, $R_2$, $R_3$ and $R_4$ are as defined above,

which is subjected, in a suitable solvent, preferably under an inert atmosphere and in the presence of n-butyllithium in hexane, to the action of a dienic aldehyde having blocked stereochemistry in the form of a tricarbonyl iron complex of the formula (VII) in accordance with the reaction described by WITTIG (Ber. 1954, 87, 1318 and Ber. 1955, 88, 1654):

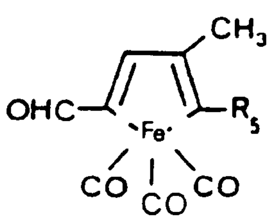

(VII)

in which $R_5$ is as defined in formula (I),

at a temperature of preferably from -5°C to -30°C, preferably from -15°C to -20°C, to yield, after extracting with a suitable organic solvent, washing and evaporating the organic phase, an intermediate complex which, after being purified by chromatography, is subjected, at the reflux temperature of the organic solvent chosen for this stage, to the action of trimethylamine N-oxide to yield, after cooling, extracting with a suitable organic solvent, washing and evaporating the organic phase, and column chromatography,

a compound of the formula (I),

which may, if desired,

- either, when $R_5$ represents a carboxy group, be converted into a salt using a pharmaceutically acceptable base,
- or be separated into its stereoisomers by a crystallisation or chromatographic technique and then, if desired, when $R_5$ represents a carboxy group, be converted into a salt using a pharmaceutically acceptable base.

8. Preparation process according to claim 7, characterised in that a compound of the formula (II):

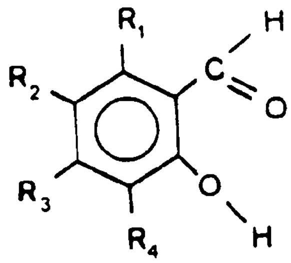

(II)

in which $R_1$, $R_2$, $R_3$ and $R_4$ are as defined in formula (I), is condensed by heating at the reflux temperature of the solvent

with 3-oxo-1-butene of the formula (III):

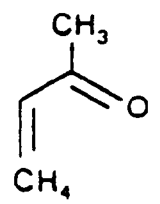

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from ketone solvents, such as 2-butanone, or amide solvents, such as dimethylformamide, to yield,

after cooling and optionally evaporating the reaction medium, taking up in water to which an alkaline agent, such as sodium hydroxide, has optionally been added, extracting with a suitable organic solvent, such as diethyl ether, chloroform or methylene chloride, washing the organic phase, evaporating the solvent and purifying the residue by crystallisation, or distillation, or chromatography on a silica or aluminium oxide column,

a compound of the formula (IV):

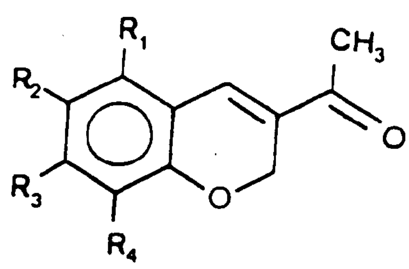

(IV)

in which $R_1$, $R_2$, $R_3$ and $R_4$ are as defined in formula (I), which is subjected to the action of ethyl bromoacetate in the presence of zinc, followed by hydrolysis of the reaction mixture when required, extraction with a suitable organic solvent preferably selected from ethyl acetate, methylene chloride, chloroform and diethyl ether, and then to the action of phosphorus oxychloride in a suitable organic solvent, evaporation of the reaction medium and fresh extraction with a solvent preferably selected from ethyl acetate, methylene chloride, chloroform and diethyl ether, to yield, after optional silica gel chromatography, a compound of the formula (VIII):

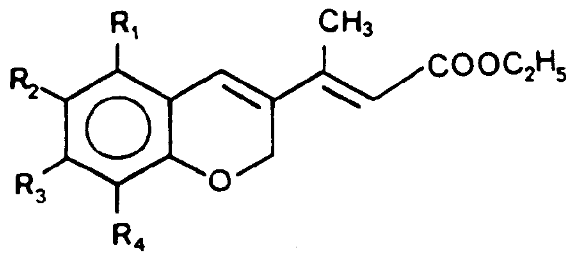

(VIII)

in which $R_1$, $R_2$, $R_3$ and $R_4$ are as defined in formula (I),

which is subjected, at a low temperature of preferably from -50 to 0°C, preferably from -25 to 5°C, in a solvent preferably selected from diethyl ether, diisopropyl ether and tetrahydrofuran, to the action of lithium aluminium hydride to yield a compound of the formula (IX):

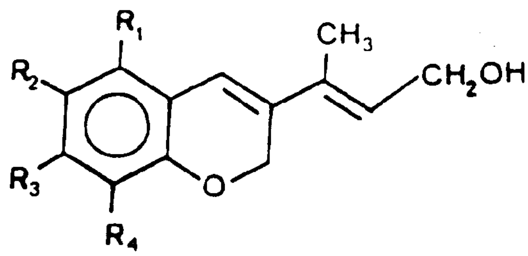

(IX)

in which $R_1$, $R_2$, $R_3$ and $R_4$ are as defined in formula (I),

which is subjected to oxidation with manganese dioxide to yield, after chromatography on silica gel and optional crystallisation, a compound of the formula (X):

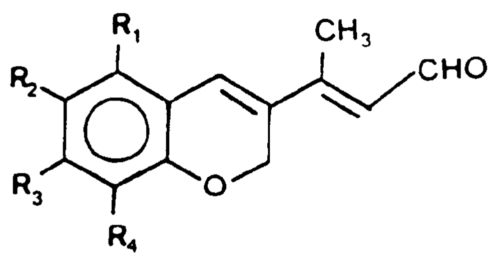

(X)

in which $R_1$, $R_2$, $R_3$ and $R_4$ are as defined in formula (I),

which is subjected to the action of a compound of the formula (XI) in accordance with the WADSWORTH-EMMONS reaction (J. Am. Chem. Soc. 1961, 83, 1733):

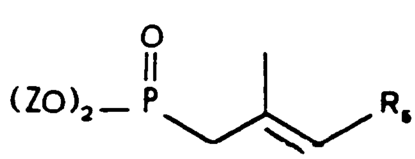

(XI)

in which $R_5$ is as defined in formula (I), and Z is a lower alkyl group,

at ambient temperature to yield, after extracting with a suitable organic solvent, evaporating, purifying by chromatography on a silica column, a compound of the formula (I),

which may, if desired,

- either, when $R_5$ represents a carboxy group, be converted into a salt using a pharmaceutically acceptable base,
- or be separated into its stereoisomers by a crystallisation or chromatographic technique and then, if

desired, when $R_5$ represents a carboxy group, be converted into a salt using a pharmaceutically acceptable base.

9. Pharmaceutical composition containing as active ingredient at least one compound according to any one of claims 1 to 6 in combination with one or more pharmaceutically acceptable, inert, non-toxic excipients or carriers.

10. Pharmaceutical composition according to claim 9 containing at least one active ingredient according to any one of claims 1 to 6, which can be used in the treatment of tumours, neoplasms and also in various skin disorders.

**Claims for the following Contracting State : ES**

1. Process for the preparation of compounds of the general formula:

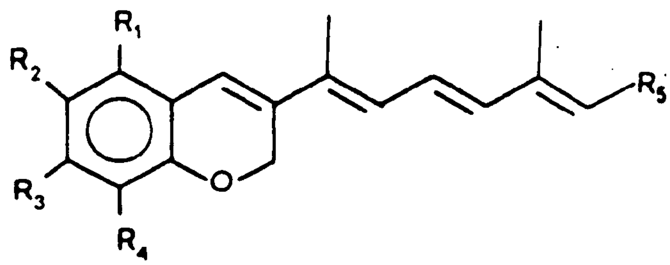

(I)

in which:

$R_1$, $R_2$, $R_3$ and $R_4$, which may be the same or different, represent a hydrogen atom, a halogen atom, a lower alkyl, lower alkenyl, lower alkoxy or lower alkenyloxy group, each of which is optionally substituted by one or more halogen atoms, and

$R_5$ represents a carboxy, lower alkoxycarbonyl, lower alkenyloxycarbonyl or lower alkynyloxycarbonyl group,

their isomers, stereoisomers, diastereoisomers and also their addition salts with a pharmaceutically acceptable base, when $R_5$ represents a carboxy group,

characterised in that a compound of the formula (II):

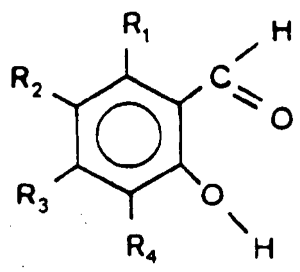

(II)

in which $R_1$, $R_2$, $R_3$ and $R_4$ are as defined in formula (I), is condensed by heating at the reflux temperature of the solvent

with 3-oxo-1-butene of the formula (III):

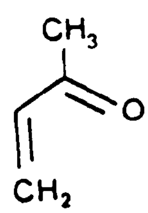

(III)

in the presence of an alkaline agent preferably selected from the alkali metal carbonates and alkaline earth

metal carbonates, or organic bases, such as, for example, triethylamine or pyridine, or a mixture made up of an alkali metal carbonate or alkaline earth metal carbonate and an organic base, in a polar solvent, preferably selected from ketone solvents, such as 2-butanone, or amide solvents, such as dimethylformamide, to yield,

after cooling and optionally evaporating the reaction medium, taking up in water to which an alkaline agent, such as sodium hydroxide, has optionally been added, extracting with a suitable organic solvent, such as diethyl ether, chloroform or methylene chloride, washing the organic phase, evaporating the solvent and purifying the residue by crystallisation, or distillation, or chromatography on a silica or aluminium oxide column,

a compound of the formula (IV):

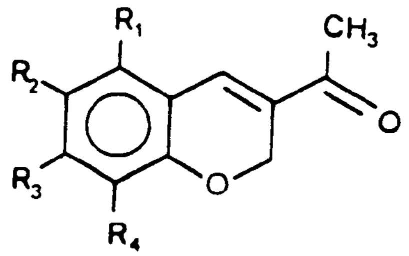

(IV)

in which $R_1$, $R_2$, $R_3$ and $R_4$ are as defined in formula (I), which, after being dissolved in a suitable organic solvent preferably selected from the lower aliphatic alcohols, is subjected, preferably in the presence of an alkaline agent, such as sodium hydroxide, to catalytic hydrogenation, preferably using a mixed alkali metal hydride, such as sodium borohydride, to yield,

after extracting with a suitable organic solvent, such as diethyl ether, dichloromethane or chloroform, optionally preceded by evaporation of the reaction medium, washing and then evaporating the organic phase and purifying, a compound of the formula (V):

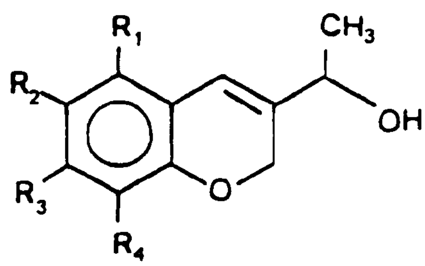

(V)

in which $R_1$, $R_2$, $R_3$ and $R_4$ are as defined above,

which is subjected to the action of a triphenylphosphonium salt in a solvent preferably selected from the lower aliphatic alcohols, at ambient temperature and with stirring, to yield, after evaporating the solvent and purifying by chromatography on silica gel and crystallisation in a suitable solvent or a mixture of suitable solvents,

a compound of the formula (VI):

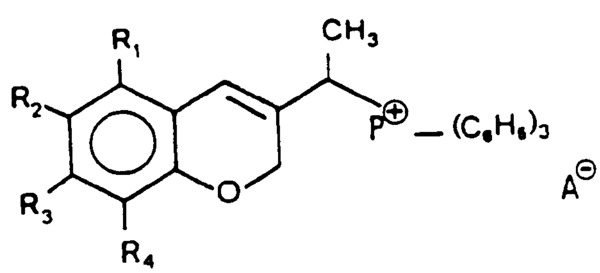

(VI)

in which $A^{\ominus}$ represents the anion of a hydracid and $R_1$, $R_2$, $R_3$ and $R_4$ are as defined above,

which is subjected, in a suitable solvent, preferably under an inert atmosphere and in the presence of n-butyllithium in hexane, to the action of a dienic aldehyde having blocked stereochemistry in the form of a tricarbonyl iron complex of the formula (VII) in accordance with the reaction described by WITTIG (Ber. 1954, 87, 1318 and Ber. 1955, 88, 1654):

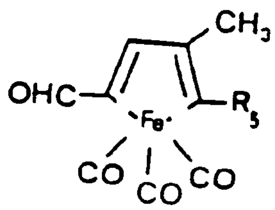

(VII)

in which $R_5$ is as defined in formula (I),

at a temperature of preferably from -5°C to -30°C, preferably from -15°C to -20°C, to yield, after extracting with a suitable organic solvent, washing and evaporating the organic phase, an intermediate complex which, after being purified by chromatography, is subjected, at the reflux temperature of the organic solvent chosen for this stage, to the action of trimethylamine N-oxide to yield, after cooling, extracting with a suitable organic solvent, washing and evaporating the organic phase, and column chromatography,

a compound of the formula (I),

which may, if desired,

- either, when $R_5$ represents a carboxy group, be converted into a salt using a pharmaceutically acceptable base,
- or be separated into its stereoisomers by a crystallisation or chromatographic technique and then, if desired, when $R_5$ represents a carboxy group, be converted into a salt using a pharmaceutically acceptable base.

**2.** Preparation process according to claim 1, characterised in that a compound of the formula (II):

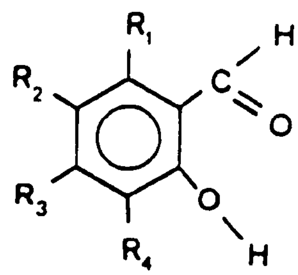

(II)

in which $R_1$, $R_2$, $R_3$ and $R_4$ are as defined in formula (I), is condensed by heating at the reflux temperature of the solvent

with 3-oxo-1-butene of the formula (III):

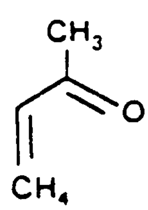

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from ketone solvents, such as 2-butanone, or amide solvents, such as dimethylformamide, to yield,

after cooling and optionally evaporating the reaction medium, taking up in water to which an alkaline agent, such as sodium hydroxide, has optionally been added, extracting with a suitable organic solvent, such as diethyl ether, chloroform or methylene chloride, washing the organic phase, evaporating the solvent and purifying the residue by crystallisation, or distillation, or chromatography on a silica or aluminium oxide column,

a compound of the formula (IV):

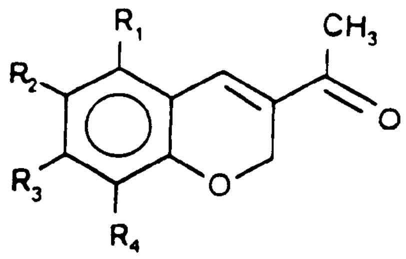

(IV)

in which $R_1$, $R_2$, $R_3$ and $R_4$ are as defined in formula (I), which is subjected to the action of ethyl bromoacetate in the presence of zinc, followed by hydrolysis of the reaction mixture when required, extraction with a suitable organic solvent preferably selected from ethyl acetate, methylene chloride, chloroform and diethyl ether, and then to the action of phosphorus oxychloride in a suitable organic solvent, evaporation of the reaction medium and fresh extraction with a solvent preferably selected from ethyl acetate, methylene chloride, chloroform and diethyl ether, to yield, after optional silica gel chromatography, a compound of the formula (VIII):

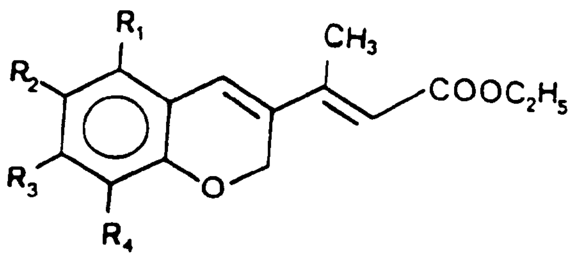

(VIII)

in which $R_1$, $R_2$, $R_3$ and $R_4$ are as defined in formula (I),

which is subjected, at a low temperature of preferably from -50 to 0°C, preferably from -25 to 5°C, in a solvent preferably selected from diethyl ether, diisopropyl ether and tetrahydrofuran, to the action of lithium aluminium hydride to yield a compound of the formula (IX):

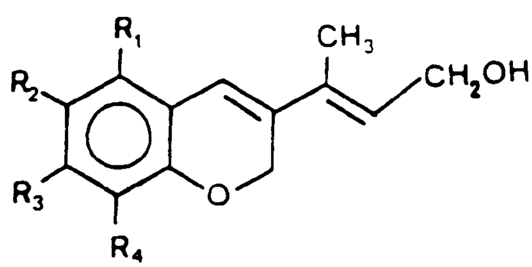

(IX)

in which $R_1$, $R_2$, $R_3$ and $R_4$ are as defined in formula (I),

which is subjected to oxidation with manganese dioxide to yield, after chromatography on silica gel and optional crystallisation, a compound of the formula (X):

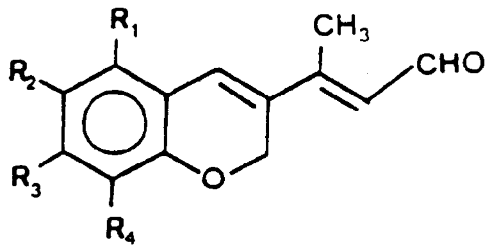

(X)

in which $R_1$, $R_2$, $R_3$ and $R_4$ are as defined in formula (I),

which is subjected to the action of a compound of the formula (XI) in accordance with the WADSWORTH-EMMONS reaction (J. Am. Chem. Soc. 1961, 83, 1733):

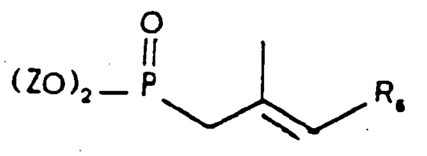

(XI)

in which $R_5$ is as defined in formula (I), and Z is a lower alkyl group,

at ambient temperature to yield, after extracting with a suitable organic solvent, evaporating, purifying by chromatography on a silica column, a compound of the formula (I),

which may, if desired,

- either, when $R_5$ represents a carboxy group, be converted into a salt using a pharmaceutically acceptable base,
- or be separated into its stereoisomers by a crystallisation or chromatographic technique and then, if desired, when $R_5$ represents a carboxy group, be converted into a salt using a pharmaceutically acceptable base.

3. Process for the preparation according to claim 1 or claim 2 of compounds of the formula (I) of which the configuration is (E,E,E).

4. Process for the preparation according to any one of claims 1, 2 and 3 of the compound that is ethyl (E,E,E)-7-(6,8-dimethyl-2H-chromen-3-yl)-3-methyl-2,4,6-octatriene carboxylate.

5. Process for the preparation according to any one of claims 1, 2 and 3 of the compound that is ethyl (E,E,E)-7-(2H-chromen-3-yl)-3-methyl-2,4,6-octatriene carboxylate.

6. Process for the preparation according to any one of claims 1, 2 and 3 of the compound that is ethyl (E,E,E)-7-(6-chloro-8-methyl-2H-chromen-3-yl)-3-methyl-2,4,6-octatriene carboxylate.

7. Process for the preparation according to any one of claims 1, 2 and 3 of the compound that is ethyl 7-(2H-chromen -3-yl)-3-methyl-2,4,6-octatriene carboxylate.

**Claims for the following Contracting State : GR**

1. Process for the preparation of compounds of the general formula:

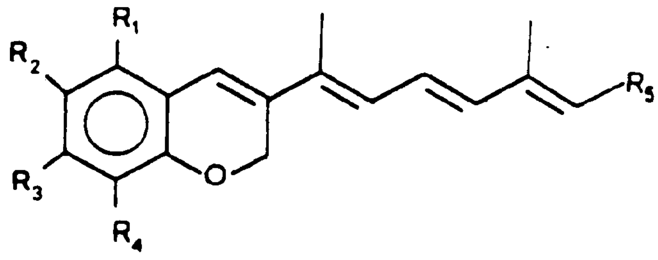

(I)

in which:

$R_1$, $R_2$, $R_3$ and $R_4$, which may be the same or different, represent a hydrogen atom, a halogen atom, a lower alkyl, lower alkenyl, lower alkoxy or lower alkenyloxy group, each of which is optionally substituted by one or more halogen atoms, and

$R_5$ represents a carboxy, lower alkoxycarbonyl, lower alkenyloxycarbonyl or lower alkynyloxycarbonyl group,

their isomers, stereoisomers, diastereoisomers and also their addition salts with a pharmaceutically acceptable base, when $R_5$ represents a carboxy group,

characterised in that a compound of the formula (II):

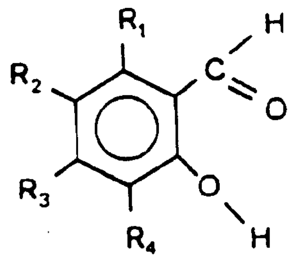

(II)

in which $R_1$, $R_2$, $R_3$ and $R_4$ are as defined in formula (I), is condensed by heating at the reflux temperature of the solvent

with 3-oxo-1-butene of the formula (III):

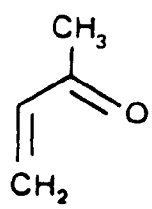

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from ketone solvents, such as 2-butanone, or amide solvents, such as dimethylformamide, to yield,

after cooling and optionally evaporating the reaction medium, taking up in water to which an alkaline agent, such as sodium hydroxide, has optionally been added, extracting with a suitable organic solvent, such as diethyl ether, chloroform or methylene chloride, washing the organic phase, evaporating the solvent and purifying the residue by crystallisation, or distillation, or chromatography on a silica or aluminium oxide column,

a compound of the formula (IV):

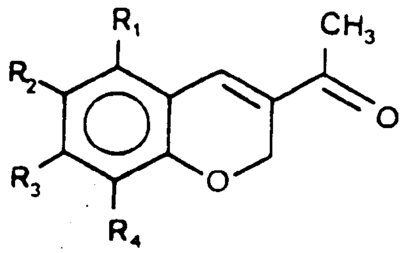

(IV)

in which $R_1$, $R_2$, $R_3$ and $R_4$ are as defined in formula (I), which, after being dissolved in a suitable organic solvent preferably selected from the lower aliphatic alcohols, is subjected, preferably in the presence of an alkaline agent, such as sodium hydroxide, to catalytic hydrogenation, preferably using a mixed alkali metal hydride, such as sodium borohydride, to yield,

after extracting with a suitable organic solvent, such as diethyl ether, dichloromethane or chloroform, optionally preceded by evaporation of the reaction medium, washing and then evaporating the organic phase and purifying, a compound of the formula (V):

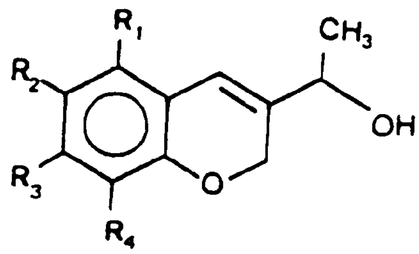

(V)

in which $R_1$, $R_2$, $R_3$ and $R_4$ are as defined above,

which is subjected to the action of a triphenylphosphonium salt in a solvent preferably selected from the lower aliphatic alcohols, at ambient temperature and with stirring, to yield, after evaporating the solvent and purifying by chromatography on silica gel and crystallisation in a suitable solvent or a mixture of suitable solvents,

a compound of the formula (VI):

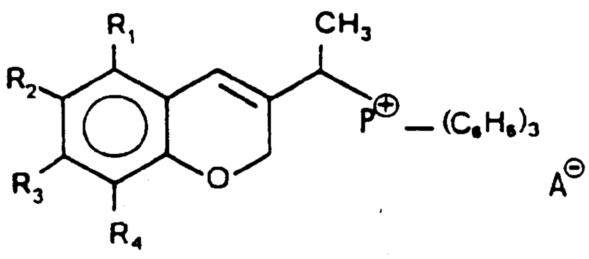

(VI)

in which $A^{\ominus}$ represents the anion of a,hydracid and $R_1$, $R_2$, $R_3$ and $R_4$ are as defined above,

which is subjected, in a suitable solvent, preferably under an inert atmosphere and in the presence of n-butyllithium in hexane, to the action of a dienic aldehyde having blocked stereochemistry in the form of a tricarbonyl iron complex of the formula (VII) in accordance with the reaction described by WITTIG (Ber. 1954, 87, 1318 and Ber. 1955, 88, 1654):

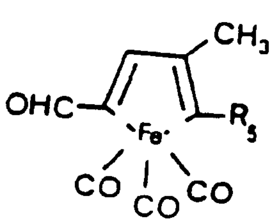

(VII)

in which $R_5$ is as defined in formula (I),

at a temperature of preferably from -5°C to -30°C, preferably from -15°C to -20°C, to yield, after extracting with a suitable organic solvent, washing and evaporating the organic phase, an intermediate complex which, after being purified by chromatography, is subjected, at the reflux temperature of the organic solvent chosen for this stage, to the action of trimethylamine N-oxide to yield, after cooling, extracting with a suitable organic solvent, washing and evaporating the organic phase, and column chromatography,

a compound of the formula (I),

which may, if desired,

- either, when $R_5$ represents a carboxy group, be converted into a salt using a pharmaceutically acceptable base,
- or be separated into its stereoisomers by a crystallisation or chromatographic technique and then, if desired, when $R_5$ represents a carboxy group, be converted into a salt using a pharmaceutically acceptable base.

**2.** Preparation process according to claim 1, characterised in that a compound of the formula (II):

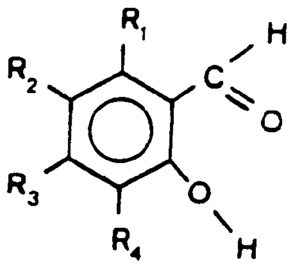

(II)

in which $R_1$, $R_2$, $R_3$ and $R_4$ are as defined in formula (I), is condensed by heating at the reflux temperature of the solvent

with 3-oxo-1-butene of the formula (III):

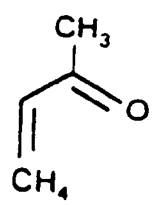

(III)

in the presence of an alkaline agent preferably selected from the alkali metal carbonates and alkaline earth metal carbonates, or organic bases, such as, for example, triethylamine or pyridine, or a mixture made up of an alkali metal carbonate or alkaline earth metal carbonate and an organic base, in a polar solvent, preferably selected from ketone solvents, such as 2-butanone, or amide solvents, such as dimethylformamide, to yield,

after cooling and optionally evaporating the reaction medium, taking up in water to which an alkaline agent, such as sodium hydroxide, has optionally been added, extracting with a suitable organic solvent, such as diethyl,ether, chloroform or methylene chloride, washing the organic phase, evaporating the solvent and purifying the residue by crystallisation, or distillation, or chromatography on a silica or aluminium oxide column,

a compound of the formula (IV):

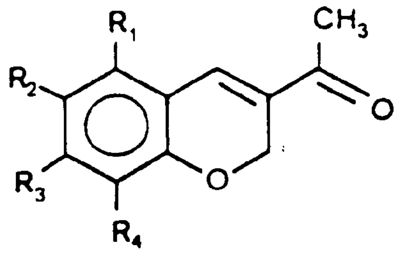

(IV)

in which $R_1$, $R_2$, $R_3$ and $R_4$ are as defined in formula (I), which is subjected to the action of ethyl bromoacetate in the presence of zinc, followed by hydrolysis of the reaction mixture when required, extraction with a suitable organic solvent preferably selected from ethyl acetate, methylene chloride, chloroform and diethyl ether, and then to the action of phosphorus oxychloride in a suitable organic solvent, evaporation of the reaction medium and fresh extraction with a solvent preferably selected from ethyl acetate, methylene chloride, chloroform and diethyl ether, to yield, after optional silica gel chromatography, a compound of the formula (VIII):

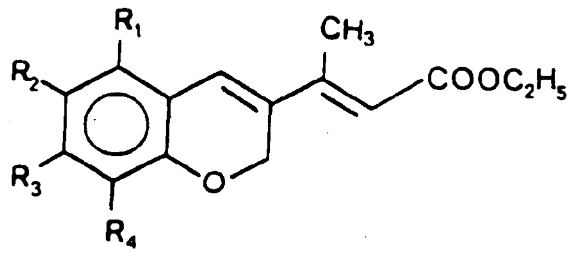

(VIII)

in which $R_1$, $R_2$, $R_3$ and $R_4$ are as defined in formula (I),

which is subjected, at a low temperature of preferably from -50 to 0°C, preferably from -25 to 5°C, in a solvent preferably selected from diethyl ether, diisopropyl ether and tetrahydrofuran, to the action of lithium aluminium hydride to yield a compound of the formula (IX):

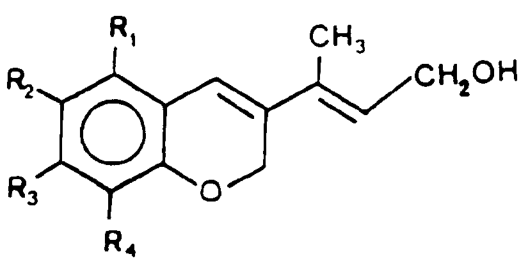

(IX)

in which $R_1$, $R_2$, $R_3$ and $R_4$ are as defined in formula (I),

which is subjected to oxidation with manganese dioxide to yield, after chromatography on silica gel and optional crystallisation, a compound of the formula (X):

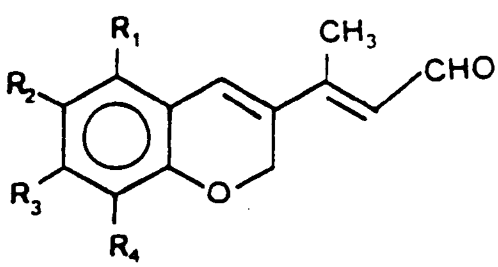

(X)

in which $R_1$, $R_2$, $R_3$ and $R_4$ are as defined in formula (I),

which is subjected to the action of a compound of the formula (XI) in accordance with the WADSWORTH-EMMONS reaction (J. Am. Chem. Soc. 1961, 83, 1733):

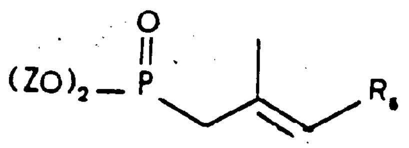 (XI)

in which $R_5$ is as defined in formula (I), and Z is a lower alkyl group,

at ambient temperature to yield, after extracting with a suitable organic solvent, evaporating, purifying by chromatography on a silica column, a compound of the formula (I),

which may, if desired,

- either, when $R_5$ represents a carboxy group, be converted into a salt using a pharmaceutically acceptable base,
- or be separated into its stereoisomers by a crystallisation or chromatographic technique and then, if desired, when $R_5$ represents a carboxy group, be converted into a salt using a pharmaceutically acceptable base.

3. Process for the preparation according to claim 1 or claim 2 of compounds of the formula (I) of which the configuration is (E,E,E).

4. Process for the preparation according to any one of claims 1, 2 and 3 of the compound that is ethyl (E,E,E)-7-(6,8-dimethyl-2H-chromen-3-yl)-3-methyl-2,4,6-octatriene carboxylate.

5. Process for the preparation according to any one of claims 1, 2 and 3 of the compound that is ethyl (E,E,E)-7-(2H-chromen-3-yl)-3-methyl-2,4,6-octatriene carboxylate.

6. Process for the preparation according to any one of claims 1, 2 and 3 of the compound that is ethyl (E,E,E)-7-(6-chloro-8-methyl-2H-chromen-3-yl)-3-methyl-2,4,6-octatriene carboxylate.

7. Process for the preparation according to any one of claims 1, 2 and 3 of the compound that is ethyl 7-(2H-chromen -3-yl)-3-methyl-2,4,6-octatriene carboxylate.